# EUROPEAN PATENT SPECIFICATION

(11) **EP 3 570 287 B1**
(45) Date of publication and mention of the grant of the patent: **18.11.2026**
(21) Application number: 19171211.6
(22) Date of filing: 25.04.2019
(51) Int. Cl.: G16H 20/30, A61B 5/318

(54) **WEARABLE MEDICAL (WM) SYSTEM MONITORING ECG SIGNAL OF AMBULATORY PATIENT FOR HEART CONDITION**
MEDIZINISCHES WEARABLE-SYSTEM ZUR ÜBERWACHUNG DES EKG-SIGNALS EINES AMBULANTEN PATIENTEN FÜR HERZERKRANKUNGEN
SYSTÈME MÉDICAL PORTABLE (WM) SURVEILLANT UN SIGNAL ECG DE D'UN PATIENT AMBULATOIRE ATTEINT D'UNE MALADIE CARDIAQUE

(30) Priority: 26.04.2018 US 201862662905 P; 23.04.2019 US 201916392541
(43) Date of publication of application: 20.11.2019
(73) Proprietor: West Affum Holdings DAC, Dublin 2 (IE)
(72) Inventor: KIM, Jaeho, KYI-1104 Grand Cayman (KY); BRESKE, Pamela, KYI-1104 Grand Cayman (KY); KAVOUNAS, Gregory T., KYI-1104 Grand Cayman (KY)
(74) Representative: Prinz & Partner mbB

(56) References cited:
- EP-A1- 0 835 635
- CN-B- 102 835 954
- JP-A- 2016 059 770
- US-A- 5 217 021
- US-A- 5 817 027
- US-A1- 2004 059 237
- US-A1- 2012 157 818
- MASON ET AL: "Electrocardiographic reference ranges derived from 79,743 ambulatory subjects", JOURNAL OF ELECTROCARDIOLOGY. ELSEVIER SCIENCE, XX, vol. 40, no. 3, 2 May 2007 (2007-05-02), pages 228 - 234.e8, XP022056401, ISSN: 0022-0736, [retrieved on 20070502], DOI: 10.1016/J.JELECTROCARD.2006.09.003

## Description

### BACKGROUND

When people suffer from some types of heart arrhythmias, the result may be that blood flow to various parts of the body is reduced. Some arrhythmias may even result in a Sudden Cardiac Arrest (SCA). SCA can lead to death very quickly, e.g. within 10 minutes, unless treated in the interim. Some observers have thought that SCA is the same as a heart attack, which it is not.

Some people have an increased risk of SCA. Such people include patients who have had a heart attack, or a prior SCA episode. A frequent recommendation for these people is to receive an Implantable Cardioverter Defibrillator (ICD). The ICD is surgically implanted in the chest, and continuously monitors the patient's electrocardiogram (ECG). If certain types of heart arrhythmias are detected, then the ICD delivers an electric shock through the heart.

People who have been identified to have an increased risk of an SCA are sometimes given a Wearable Cardioverter Defibrillator (WCD) system, to wear until the time that their ICD is implanted. Early versions of such systems were called wearable cardiac defibrillator systems. A WCD system typically includes a harness, vest, belt, or other garment that the patient is to wear. The WCD system further includes electronic components, such as a defibrillator and electrodes, coupled to the harness, vest, or other garment. When the patient wears the WCD system, the electrodes may make good electrical contact with the patient's skin, and therefore can help sense the patient's ECG. If a shockable heart arrhythmia is detected from the ECG, then the defibrillator delivers an appropriate electric shock through the patient's body, and thus through the heart.

This may restart the patient's heart and thus save their life.

US 2004/059237 A1 discloses a wearable medical system for ECG connected to a processor which compares the sensed waveform to a reference and in case of deviations from the norm issues an alert.

This system does not calculate similarity metrics between the sensed waveform and the reference waveform, and does not include mechanical biasing of the electrodes.

US 2014/0364918 A1 discloses a wearable medical system for an ambulatory patient with electrocardiogram electrodes and a support structure configured to be worn by the patient. The wearable medical system further comprises an output device and a memory configured to store a reference. The wearable medical system also comprises a processor which is configured to compare a test value with a reference value and output an alert responsive to the difference exceeding an alert threshold.

US 5 217 021 A1 discloses a cardiac control and monitoring device which is able to process cardiac electrical data in compressed form while detecting abnormal ventricular signals.

CN 1028359504 A discloses the creation of a waveform template for detecting a ventricular premature beat with a higher accuracy and less calculation effort.

JP 2016 059770 A discloses a method for analyzing EKG signals and detecting ventricular premature contractions by creating a template and calculating a correlation coefficient based on the similarity of a test signal and the template and a similarity of amplitudes.

US 2012 / 0 157 818 A1 discloses an electrically non-conductive back plate for supporting the back of a patient in a supine position which comprises on its front face facing the back of the patient a pair or more of ECG electrodes capable of maintaining electrically conductive skin contact with the patient's back.

All subject matter discussed in this Background section of this document is not necessarily prior art, and may not be presumed to be prior art simply because it is presented in this Background section. Plus, any reference to any prior art in this description is not, and should not be taken as, an acknowledgement or any form of suggestion that such prior art forms parts of the common general knowledge in any art in any country. Along these lines, any recognition of problems in the prior art discussed in this Background section or associated with such subject matter should not be treated as prior art, unless expressly stated to be prior art. Rather, the discussion of any subject matter in this Background section should be treated as part of the approach taken towards the particular problem by the inventors. This approach in and of itself may also be inventive.

### BRIEF SUMMARY

The invention is defined by the appended claims.

### BRIEF DESCRIPTION OF THE DRAWINGS

FIG. 1 is a diagram of components of a sample wearable cardioverter defibrillator (WCD) system, made according to embodiments.
FIG. 2 is a diagram showing sample components of an external defibrillator, such as the one belonging in the system of FIG. 1, and which is made according to embodiments.
FIG. 3A is a diagram showing an electrode, such as a possible embodiment of an electrode of FIG. 1, which is not being biased towards the body of the wearer according to embodiments.
FIG. 3B is a diagram showing the electrode of FIG. 3A, but in which the electrode is instead being biased towards the body of the wearer according to embodiments.
FIG. 4 is a diagram of sample embodiments of components of a WCD system.
FIG. 5 is a conceptual diagram for illustrating an example how multiple electrodes may be used for sensing ECG signals along different vectors in a WCD system according to embodiments.
FIG. 6 is a diagram of components of a WM system made according to embodiments.
FIG. 7 shows multiple related diagrams, including time diagrams of a patient's early ECG signals that are used for creating a reference template, and of later, test ECG signals that are compared against the reference template, and also for conditions for selecting waveform segments to generate the reference template, and conditions for later causing the comparison, made according to embodiments.
FIG. 8 is a time diagram of a segment of a waveform amplitude of an ideal ECG signal, further indicating aspects that can be monitored by embodiments.
FIG. 9 is a time diagram of a sample ECG waveform that has been filtered so that it can be aligned with others according to embodiments.
FIG. 10 is a flowchart for illustrating additional sample methods for constructing a reference template according to embodiments.
FIG. 11 is a flowchart for illustrating sample methods according to embodiments.
FIG. 12 shows sample time diagrams of a healthy and of suspect ECG waveforms that can be detected by embodiments.
FIG. 13 shows a sample equation for computing a waveform similarity coefficient according to embodiments, for detecting suspect ECG waveforms such as some of the waveforms in FIG. 12.
FIG. 14A shows a first time diagram of an ECG signal with detected QRS peaks, and a second time diagram showing computed waveform similarity coefficients for the peaks, according to embodiments.
FIG. 14B shows aggregated signals that could be made from the ECG signals of FIG. 14A, according to embodiments.
FIG. 15A shows a first time diagram of an ECG signal with detected QRS peaks, and a second time diagram showing computed waveform similarity coefficients for the peaks, according to embodiments.
FIG. 15B shows aggregated signals that could be made from the ECG signals of FIG. 15A, according to embodiments.
FIG. 16 is a flowchart for illustrating sample methods according to embodiments.

### DETAILED DESCRIPTION

As has been mentioned, the present description is about wearable medical (WM) systems, devices, storage media that may store programs, and methods. Embodiments are now described in more detail.

A wearable cardioverter defibrillator (WCD) system according to embodiments may protect an ambulatory patient by electrically restarting their heart if needed. Such a WCD system may have a number of components. These components can be provided separately as modules that can be interconnected, or can be combined with other components, and so on.

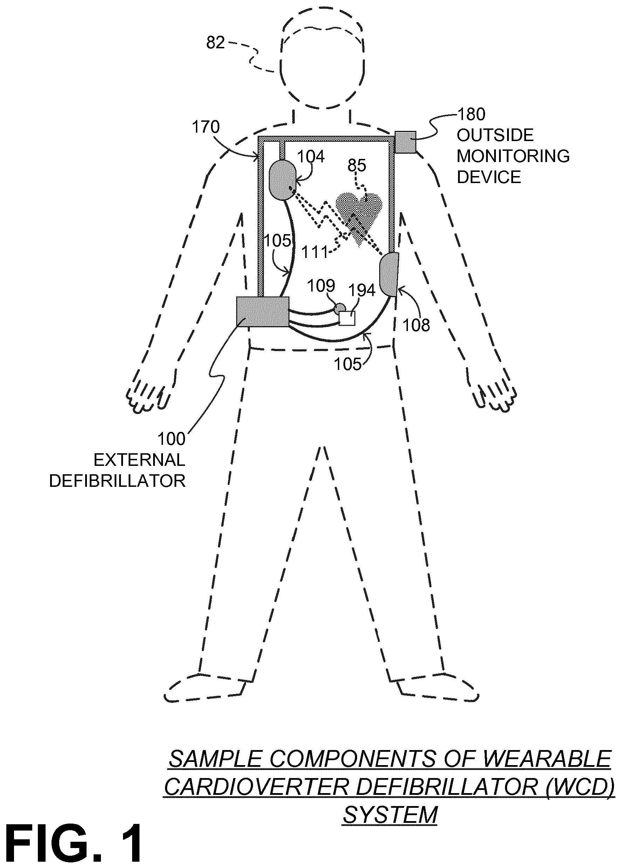

FIG. 1 depicts a patient 82. Patient 82 may also be referred to as a person and/or wearer, since the patient is wearing components of the WCD system. Patient 82 is ambulatory, which means that, while wearing the wearable portion of the WCD system, patient 82 can walk around and is not necessarily bed-ridden. While patient 82 may be considered to be also a "user" of the WCD system, this is not a requirement. For instance, a user of the wearable cardioverter defibrillator (WCD) may also be a clinician such as a doctor, nurse, emergency medical technician (EMT) or other similarly tasked individual or group of individuals. In some cases, a user may even be a bystander. The particular context of these and other related terms within this description should be interpreted accordingly.

A WCD system according to embodiments can be configured to defibrillate the patient who is wearing the designated parts the WCD system. Defibrillating can be by the WCD system delivering an electrical charge to the patient's body in the form of an electric shock. The electric shock can be delivered in one or more pulses.

In particular, FIG. 1 also depicts components of a WCD system made according to embodiments. One such component is a support structure 170 that is wearable by ambulatory patient 82. Accordingly, support structure 170 is configured to be worn by ambulatory patient 82 for at least several hours per day, and for at least several days, even a few months. It will be understood that support structure 170 is shown only generically in FIG. 1, and in fact partly conceptually. FIG. 1 is provided merely to illustrate concepts about support structure 170, and is not to be construed as limiting how support structure 170 is implemented, or how it is worn.

Support structure 170 can be implemented in many different ways. For example, it can be implemented in a single component or a combination of multiple components. In embodiments, support structure 170 could include a vest, a half-vest, a garment, etc. In such embodiments such items can be worn similarly to analogous articles of clothing. In embodiments, support structure 170 could include a harness, one or more belts or straps, etc. In such embodiments, such items can be worn by the patient around the torso, hips, over the shoulder, etc. In embodiments, support structure 170 can include a container or housing, which can even be waterproof. In such embodiments, the support structure can be worn by being attached to the patient's body by adhesive material, for example as shown and described in US Patent No. 8,024,037. Support structure 170 can even be implemented as described for the support structure of US Pat. App. No. US2017/0056682. Of course, in such embodiments, the person skilled in the art will recognize that additional components of the WCD system can be in the housing of a support structure instead of being attached externally to the support structure, for example as described in the US2017/0056682 document. There can be other examples.

FIG. 1 shows a sample external defibrillator 100. As described in more detail later in this document, some aspects of external defibrillator 100 include a housing and an energy storage module within the housing. As such, in the context of a WCD system, defibrillator 100 is sometimes called a main electronics module. The energy storage module can be configured to store an electrical charge. Other components can cause at least some of the stored electrical charge to be discharged via electrodes through the patient, so as to deliver one or more defibrillation shocks through the patient.

FIG. 1 also shows sample defibrillation electrodes 104, 108, which are coupled to external defibrillator 100 via electrode leads 105. Defibrillation electrodes 104, 108 can be configured to be worn by patient 82 in a number of ways. For instance, defibrillator 100 and defibrillation electrodes 104, 108 can be coupled to support structure 170, directly or indirectly. In other words, support structure 170 can be configured to be worn by ambulatory patient 82 so as to maintain at least one of electrodes 104, 108 on the body of ambulatory patient 82, while patient 82 is moving around, etc. The electrode can be thus maintained on the body by being attached to the skin of patient 82, simply pressed against the skin directly or through garments, etc. In some embodiments the electrode is not necessarily pressed against the skin, but becomes biased that way upon sensing a condition that could merit intervention by the WCD system. In addition, many of the components of defibrillator 100 can be considered coupled to support structure 170 directly, or indirectly via at least one of defibrillation electrodes 104, 108.

When defibrillation electrodes 104, 108 make good electrical contact with the body of patient 82, defibrillator 100 can administer, via electrodes 104, 108, a brief, strong electric pulse 111 through the body. Pulse 111 is also known as shock, defibrillation shock, therapy, electrotherapy, therapy shock, etc. Pulse 111 is intended to go through and restart heart 85, in an effort to save the life of patient 82. Pulse 111 can further include one or more pacing pulses of lesser magnitude to simply pace heart 85 if needed, and so on.

A prior art defibrillator typically decides whether to defibrillate or not based on an ECG signal of the patient. However, external defibrillator 100 may initiate defibrillation, or hold-off defibrillation, based on a variety of inputs, with the ECG signal merely being one of these inputs. In this example, ECG electrodes are provided, of which ECG electrode 109 is shown. In addition, in this example, a biasing mechanism 194 is provided for biasing ECG electrode 109. More about such biasing mechanisms is described later in this document.

A WCD system according to embodiments can obtain data from patient 82. For collecting such data, the WCD system may optionally include at least an outside monitoring device 180. Device 180 is called an "outside" device because it could be provided as a standalone device, for example not within the housing of defibrillator 100. Device 180 can be configured to sense or monitor at least one local parameter. A local parameter can be a parameter of patient 82, or a parameter of the WCD system, or a parameter of the environment, as will be described later in this document.

For some of these parameters, device 180 may include one or more sensors or transducers. Each one of such sensors can be configured to sense a parameter of patient 82, and to render an input responsive to the sensed parameter. In some embodiments the input is quantitative, such as values of a sensed parameter; in other embodiments the input is qualitative, such as informing whether or not a threshold is crossed, and so on. Sometimes these inputs about patient 82 are also called physiological inputs and patient inputs. In embodiments, a sensor can be construed more broadly, as encompassing many individual sensors.

Optionally, device 180 is physically coupled to support structure 170. In addition, device 180 may be communicatively coupled with other components that are coupled to support structure 170. Such communication can be implemented by a communication module, as will be deemed applicable by a person skilled in the art in view of this description.

In embodiments, one or more of the components of the shown WCD system may be customized for patient 82. This customization may include a number of aspects. For instance, support structure 170 can be fitted to the body of patient 82. For another instance, baseline physiological parameters of patient 82 can be measured, such as the heart rate of patient 82 while resting, while walking, motion detector outputs while walking, etc. The measured values of such baseline physiological parameters can be used to customize the WCD system, in order to make its diagnoses more accurate, since patients' bodies differ from one another. Of course, such parameter values can be stored in a memory of the WCD system, and so on. Moreover, a programming interface can be made according to embodiments, which receives such measured values of baseline physiological parameters. Such a programming interface may input automatically in the WCD system these, along with other data.

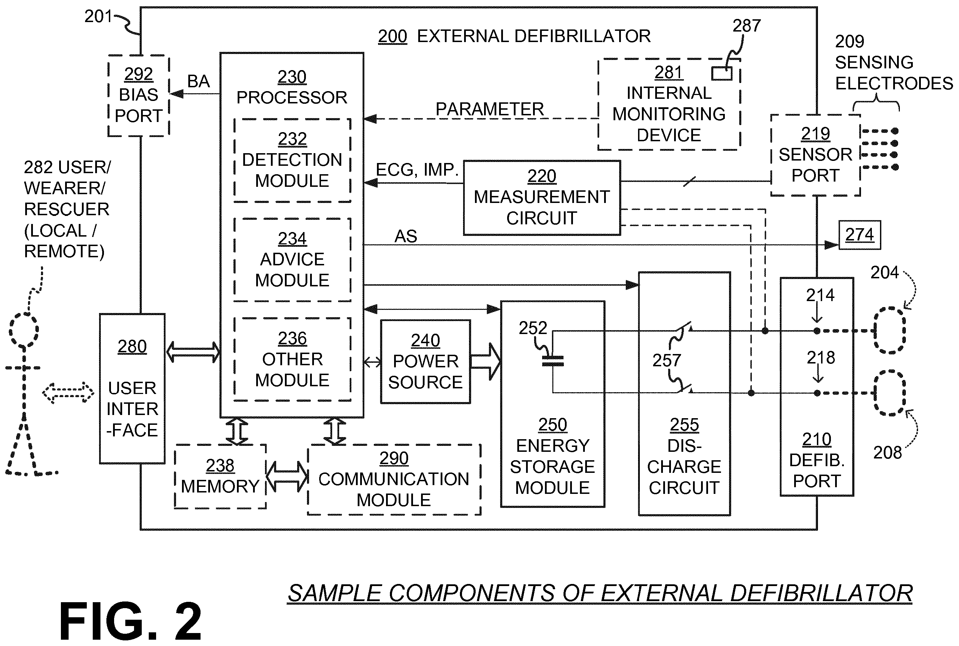

FIG. 2 is a diagram showing components of an external defibrillator 200, made according to embodiments. These components can be, for example, included in external defibrillator 100 of FIG. 1. The components shown in FIG. 2 can be provided in a housing 201, which may also be referred to as casing 201.

External defibrillator 200 is intended for a patient who would be wearing it, such as ambulatory patient 82 of FIG. 1. Defibrillator 200 may further include a user interface 280 for a user 282. User 282 can be patient 82, also known as wearer 82. Or, user 282 can be a local rescuer at the scene, such as a bystander who might offer assistance, or a trained person. Or, user 282 might be a remotely located trained caregiver in communication with the WCD system.

User interface 280 can be made in a number of ways. User interface 280 may include output devices, which can be visual, audible or tactile, for communicating to a user by outputting images, sounds or vibrations. Images, sounds, vibrations, and anything that can be perceived by user 282 can also be called human-perceptible indications (HPIs). There are many examples of output devices. For example, an output device can be a light, or a screen to display what is sensed, detected and/or measured, and provide visual feedback to rescuer 282 for their resuscitation attempts, and so on. Another output device can be a speaker, which can be configured to issue voice prompts, beeps, loud alarm sounds and/or words to warn bystanders, etc.

User interface 280 may further include input devices for receiving inputs from users. Such input devices may include various controls, such as pushbuttons, keyboards, touchscreens, one or more microphones, and so on. An input device can be a cancel switch, which is sometimes called an "I am alive" switch or "live man" switch. In some embodiments, actuating the cancel switch can prevent the impending delivery of a shock.

Defibrillator 200 may include an internal monitoring device 281. Device 281 is called an "internal" device because it is incorporated within housing 201. Monitoring device 281 can sense or monitor patient parameters such as patient physiological parameters, system parameters and/or environmental parameters, all of which can be called patient data. In other words, internal monitoring device 281 can be complementary or an alternative to outside monitoring device 180 of FIG. 1. Allocating which of the parameters are to be monitored by which of monitoring devices 180, 281 can be done according to design considerations. Device 281 may include one or more sensors, as also described elsewhere in this document.

Patient parameters may include patient physiological parameters. Patient physiological parameters may include, for example and without limitation, those physiological parameters that can be of any help in detecting by the WCD system whether or not the patient is in need of a shock or other intervention or assistance. Patient physiological parameters may also optionally include the patient's medical history, event history and so on. Examples of such parameters include the patient's ECG, heart rate, blood oxygen level, blood flow, blood pressure, blood perfusion, pulsatile change in light transmission or reflection properties of perfused tissue, heart sounds, heart wall motion, breathing sounds and pulse. Accordingly, monitoring devices 180, 281 may include one or more sensors configured to acquire patient physiological signals. Examples of such sensors or transducers include one or more electrodes to detect ECG data, a perfusion sensor, a pulse oximeter, a device for detecting blood flow (e.g. a Doppler device), a sensor for detecting blood pressure (e.g. a cuff), an optical sensor, illumination detectors and sensors perhaps working together with light sources for detecting color change in tissue, a motion sensor, a device that can detect heart wall movement, a sound sensor, a device with a microphone, an SpO₂ sensor, and so on. In view of this disclosure, it will be appreciated that such sensors can help detect the patient's pulse, and can therefore also be called pulse detection sensors, pulse sensors, and pulse rate sensors, heart rate sensors, heart beat irregularity sensors, etc. In addition, a person skilled in the art may implement other ways of performing pulse detection.

In some embodiments, the local parameter is a trend that can be detected in a monitored physiological parameter of patient 282. A trend can be detected by comparing values of parameters at different times over short and long terms. Parameters whose detected trends can particularly help a cardiac rehabilitation program include: a) cardiac function (e.g. ejection fraction, stroke volume, cardiac output, etc.); b) heart rate variability at rest or during exercise; c) heart rate profile during exercise and measurement of activity vigor, such as from the profile of an accelerometer signal and informed from adaptive rate pacemaker technology; d) heart rate trending; e) perfusion, such as from SpO₂, CO₂, or other parameters such as those mentioned above, f) respiratory function, respiratory rate, etc.; g) motion, level of activity; and so on. Once a trend is detected, it can be stored and/or reported via a communication link, along perhaps with a warning if warranted. From the report, a physician monitoring the progress of patient 282 will know about a condition that is either not improving or deteriorating.

Patient state parameters include recorded aspects of patient 282, such as motion, posture, whether they have spoken recently plus maybe also what they said, and so on, plus optionally the history of these parameters. Or, one of these monitoring devices could include a location sensor such as a Global Positioning System (GPS) location sensor. Such a sensor can detect the location, plus a speed can be detected as a rate of change of location over time. Many motion detectors output a motion signal that is indicative of the motion of the detector, and thus of the patient's body. Patient state parameters can be very helpful in narrowing down the determination of whether SCA is indeed taking place.

A WCD system made according to embodiments may thus include a motion detector. In embodiments, a motion detector can be implemented within monitoring device 180 or monitoring device 281. Such a motion detector can be made in many ways as is known in the art, for example by using an accelerometer. In this example, a motion detector 287 is implemented within monitoring device 281. A motion detector of a WCD system according to embodiments can be configured to detect a motion event. A motion event can be defined as is convenient, for example a change in motion from a baseline motion or rest, etc. In such cases, a sensed patient parameter is motion.

System parameters of a WCD system can include system identification, battery status, system date and time, reports of self-testing, records of data entered, records of episodes and intervention, and so on. In response to the detected motion event, the motion detector may render or generate, from the detected motion event or motion, a motion detection input that can be received by a subsequent device or functionality.

Environmental parameters can include ambient temperature and pressure. Moreover, a humidity sensor may provide information as to whether or not it is likely raining. Presumed patient location could also be considered an environmental parameter. The patient location could be presumed, if monitoring device 180 or 281 includes a GPS location sensor as per the above, and if it is presumed that the patient is wearing the WCD system.

Defibrillator 200 typically includes a defibrillation port 210, which can be a socket in housing 201. Defibrillation port 210 includes electrical nodes 214, 218. Leads of defibrillation electrodes 204, 208, such as leads 105 of FIG. 1, can be plugged into defibrillation port 210, so as to make electrical contact with nodes 214, 218, respectively. It is also possible that defibrillation electrodes 204, 208 are connected continuously to defibrillation port 210, instead. Either way, defibrillation port 210 can be used for guiding, via electrodes, to the wearer at least some of the electrical charge that has been stored in an energy storage module 250 that is described more fully later in this document. The electric charge will be the shock for defibrillation, pacing, and so on.

Defibrillator 200 may optionally also have a sensor port 219 in housing 201, which is also sometimes known as an ECG port. Sensor port 219 can be adapted for plugging in sensing electrodes 209, which are also known as ECG electrodes and ECG leads. It is also possible that sensing electrodes 209 can be connected continuously to sensor port 219, instead. Sensing electrodes 209 are types of transducers that can help sense an ECG signal, e.g. a 12-lead signal, or a signal from a different number of leads, especially if they make good electrical contact with the body of the patient and in particular with the skin of the patient. As with defibrillation electrodes 204, 208, the support structure can be configured to be worn by patient 282 so as to maintain sensing electrodes 209 on a body of patient 282. For example, sensing electrodes 209 can be attached to the inside of support structure 170 for making good electrical contact with the patient, similarly with defibrillation electrodes 204, 208.

Optionally a WCD system according to embodiments also includes a fluid that it can deploy automatically between the electrodes and the patient's skin. The fluid can be conductive, such as by including an electrolyte, for establishing a better electrical contact between the electrodes and the skin. Electrically speaking, when the fluid is deployed, the electrical impedance between each electrode and the skin is reduced. Mechanically speaking, the fluid may be in the form of a low-viscosity gel, so that it does not flow away, after being deployed, from the location it is released near the electrode. The fluid can be used for both defibrillation electrodes 204, 208, and for sensing electrodes 209.

The fluid may be initially stored in a fluid reservoir, not shown in FIG. 2. Such a fluid reservoir can be coupled to the support structure. In addition, a WCD system according to embodiments further includes a fluid deploying mechanism 274. Fluid deploying mechanism 274 can be configured to cause at least some of the fluid to be released from the reservoir, and be deployed near one or both of the patient locations to which electrodes 204, 208 are configured to be attached to the patient. In some embodiments, fluid deploying mechanism 274 is activated prior to the electrical discharge responsive to receiving activation signal AS from a processor 230, which is described more fully later in this document.

In some embodiments, defibrillator 200 also includes a measurement circuit 220, as one or more of its modules working together with its sensors or transducers. Measurement circuit 220 senses one or more electrical physiological signals of the patient from sensor port 219, if provided. Even if defibrillator 200 lacks sensor port 219, measurement circuit 220 may optionally obtain physiological signals through nodes 214, 218 instead, when defibrillation electrodes 204, 208 are attached to the patient. In these cases, the input reflects an ECG measurement. The patient parameter can be an ECG, which can be sensed as a voltage difference between electrodes 204, 208. In addition, the patient parameter can be an impedance, which can be sensed between electrodes 204, 208 and/or between the connections of sensor port 219 considered pairwise. Sensing the impedance can be useful for detecting, among other things, whether these electrodes 204, 208 and/or sensing electrodes 209 are not making good electrical contact with the patient's body. These patient physiological signals may be sensed when available. Measurement circuit 220 can then render or generate information about them as inputs, data, other signals, etc. As such, measurement circuit 220 can be configured to render a patient input responsive to a patient parameter sensed by a sensor. In some embodiments, measurement circuit 220 can be configured to render a patient input, such as values of an ECG signal, responsive to the ECG signal sensed by sensing electrodes 209. More strictly speaking, the information rendered by measurement circuit 220 is output from it, but this information can be called an input because it is received as an input by a subsequent device or functionality.

Defibrillator 200 also includes a processor 230. Processor 230 may be implemented in a number of ways. Such ways include, by way of example and not of limitation, digital and/or analog processors such as microprocessors and Digital Signal Processors (DSPs), controllers such as microcontrollers, software running in a machine, programmable circuits such as Field Programmable Gate Arrays (FPGAs), Field-Programmable Analog Arrays (FPAAs), Programmable Logic Devices (PLDs), Application Specific Integrated Circuits (ASICs), any combination of one or more of these, and so on.

Processor 230 may include, or have access to, a non-transitory storage medium, such as memory 238 that is described more fully later in this document. Such a memory can have a non-volatile component for storage of machine-readable and machine-executable instructions. A set of such instructions can also be called a program. The instructions, which may also be referred to as "software," generally provide functionality by performing acts, operations and/or methods as may be disclosed herein or understood by one skilled in the art in view of the disclosed embodiments. In some embodiments, and as a matter of convention used herein, instances of the software may be referred to as a "module" and by other similar terms. Generally, a module includes a set of the instructions so as to offer or fulfill a particular functionality. Embodiments of modules and the functionality delivered are not limited by the embodiments described in this document.

Processor 230 can be considered to have a number of modules. One such module can be a detection module 232. Detection module 232 can include a Ventricular Fibrillation (VF) detector. The patient's sensed ECG from measurement circuit 220, which can be available as inputs, data that reflect values, or values of other signals, may be used by the VF detector to determine whether the patient is experiencing VF. Detecting VF is useful, because VF typically results in SCA. Detection module 232 can also include a Ventricular Tachycardia (VT) detector for detecting VT, and so on.

Another such module in processor 230 can be an advice module 234, which generates advice for what to do. The advice can be based on outputs of detection module 232. There can be many types of advice according to embodiments. In some embodiments, the advice is a shock/no shock determination that processor 230 can make, for example via advice module 234. The shock/no shock determination can be made by executing a stored Shock Advisory Algorithm. A Shock Advisory Algorithm can make a shock/no shock determination from one or more ECG signals that are captured according to embodiments, and determine whether or not a shock criterion is met. The determination can be made from a rhythm analysis of the captured ECG signal or otherwise. For example, there can be shock decisions for VF, VT, etc.

One more example of a decision that can be made is to bias one or more electrodes towards the person's body, as will be described later in this document. The decision can be communicated in some of these embodiments by generating a biasing signal BA. Defibrillator 300 optionally includes also a bias port 292 for exporting biasing signal BA from bias port 292 to a biasing mechanism of the system, such as biasing mechanism 194.

In some embodiments, when the determination is to shock, an electrical charge is delivered to the patient. Delivering the electrical charge is also known as discharging and shocking the patient. As mentioned above, such can be for defibrillation, pacing, and so on.

In perfect conditions, a very reliable shock/no shock determination can be made from a segment of the sensed ECG signal of the patient. In practice, however, the ECG signal is often corrupted by electrical noise, which makes it difficult to analyze. Too much noise sometimes causes an incorrect detection of a heart arrhythmia, resulting in a false alarm to the patient. Noisy ECG signals may be handled as described in US patent application Ser. No. 16/037,990, filed on July 17, 2018 and since published as US 2019/0030351 A1, and also in US patent application Ser. No. 16/038,007, filed on July 17, 2018 and since published as US 2019/0030352 A1, both by the same applicant.

Processor 230 can include additional modules, such as other module 236, for other functions. In addition, if internal monitoring device 281 is indeed provided, processor 230 may receive its inputs, etc.

Defibrillator 200 optionally further includes a memory 238, which can work together with processor 230. Memory 238 may be implemented in a number of ways. Such ways include, by way of example and not of limitation, volatile memories, Nonvolatile Memories (NVM), Read-Only Memories (ROM), Random Access Memories (RAM), magnetic disk storage media, optical storage media, smart cards, flash memory devices, any combination of these, and so on. Memory 238 is thus a non-transitory storage medium. Memory 238, if provided, can include programs for processor 230, which processor 230 may be able to read and execute. More particularly, the programs can include sets of instructions in the form of code, which processor 230 may be able to execute upon reading. Executing is performed by physical manipulations of physical quantities, and may result in functions, operations, processes, acts, actions and/or methods to be performed, and/or the processor to cause other devices or components or blocks to perform such functions, operations, processes, acts, actions and/or methods. The programs can be operational for the inherent needs of processor 230, and can also include protocols and ways that decisions can be made by advice module 234. In addition, memory 238 can store prompts for user 282, if this user is a local rescuer. Moreover, memory 238 can store data. This data can include patient data, system data and environmental data, for example as learned by internal monitoring device 281 and outside monitoring device 180. The data can be stored in memory 238 before it is transmitted out of defibrillator 200, or be stored there after it is received by defibrillator 200.

Defibrillator 200 can optionally include a communication module 290, for establishing one or more wired or wireless communication links with other devices of other entities, such as a remote assistance center, Emergency Medical Services (EMS), and so on. The communication links can be used to transfer data and commands. The data may be patient data, event information, therapy attempted, CPR performance, system data, environmental data, and so on. For example, communication module 290 may transmit wirelessly, e.g. on a daily basis, heart rate, respiratory rate, and other vital signs data to a server accessible over the internet, for instance as described in US 20140043149. This data can be analyzed directly by the patient's physician and can also be analyzed automatically by algorithms designed to detect a developing illness and then notify medical personnel via text, email, phone, etc. Module 290 may also include such interconnected sub-components as may be deemed necessary by a person skilled in the art, for example an antenna, portions of a processor, supporting electronics, outlet for a telephone or a network cable, etc.

Defibrillator 200 may also include a power source 240. To enable portability of defibrillator 200, power source 240 typically includes a battery. Such a battery is typically implemented as a battery pack, which can be rechargeable or not. Sometimes a combination is used of rechargeable and non-rechargeable battery packs. Other embodiments of power source 240 can include an AC power override, for where AC power will be available, an energy-storing capacitor, and so on. Appropriate components may be included to provide for charging or replacing power source 240. In some embodiments, power source 240 is controlled and/or monitored by processor 230.

Defibrillator 200 may additionally include an energy storage module 250. Energy storage module 250 can be coupled to the support structure of the WCD system, for example either directly or via the electrodes and their leads. Module 250 is where some electrical energy can be stored temporarily in the form of an electrical charge, when preparing it for discharge to administer a shock. In embodiments, module 250 can be charged from power source 240 to the desired amount of energy, as controlled by processor 230. In typical implementations, module 250 includes a capacitor 252, which can be a single capacitor or a system of capacitors, and so on. In some embodiments, energy storage module 250 includes a device that exhibits high power density, such as an ultracapacitor. As described above, capacitor 252 can store the energy in the form of an electrical charge, for delivering to the patient.

A decision to shock can be made responsive to the shock criterion being met, as per the above-mentioned determination. When the decision is to shock, processor 230 can be configured to cause at least some or all of the electrical charge stored in module 250 to be discharged through patient 82 while the support structure is worn by patient 82, so as to deliver a shock 111 to patient 82.

For causing the discharge, defibrillator 200 moreover includes a discharge circuit 255. When the decision is to shock, processor 230 can be configured to control discharge circuit 255 to discharge through the patient at least some of all of the electrical charge stored in energy storage module 250. Discharging can be to nodes 214, 218, and from there to defibrillation electrodes 204, 208, so as to cause a shock to be delivered to the patient. Circuit 255 can include one or more switches 257. Switches 257 can be made in a number of ways, such as by an H-bridge, and so on. Circuit 255 could also be thus controlled via processor 230, and/or user interface 280.

A time waveform of the discharge may be controlled by thus controlling discharge circuit 255. The amount of energy of the discharge can be controlled by how much energy storage module has been charged, and also by how long discharge circuit 255 is controlled to remain open.

Defibrillator 200 can optionally include other components.

In a number of embodiments, the ECG electrodes are not always making good electrical contact with the patient's skin. In fact, a certain one of these ECG electrodes can be coupled to the support structure such that, while the support structure is worn by the patient, the certain ECG electrode is at a so-called unbiased state. When in the unbiased state, the certain ECG electrode is moveable with respect to the patient's body, for example as a result of the patient's moving around. For example, the certain ECG electrode could contact the patient's skin as a regular garment does, for example as does a shirt that is not tightened around the patient's body. As such the certain ECG electrode could shift around the patient's skin, and occasionally lose contact with it. In those occasional moments, the electrical impedance between the certain ECG electrode and the patient's skin would become infinite. Of course, what is written about the certain ECG electrode could also apply for companions electrodes that performs a similar function.

It is that lack of the certain ECG electrode's making consistently good electrical contact with the skin that makes the support structure more comfortable to wear for the long term. Of course, the certain ECG electrode not necessarily making consistently good electrical contact for the long term is not desirable, but that will be addressed by the biasing mechanism that is now described.

Embodiments of the system of the invention may additionally include a biasing mechanism. The biasing mechanism can be configured to cause the certain ECG electrode to transition from the above described unbiased state to a so-called biased state. When in the biased state, the certain ECG electrode is biased towards the patient's body against the support structure. The biasing, then, is by a force that causes the certain ECG electrode to be less moveable with respect to the patient's body than when in the unbiased state. As such, when in the biased state, the certain ECG electrode makes better and/or more reliable electrical contact with the patient's skin than in the unbiased state. The better electrical contact can be used for more reliable defibrillation and or receiving ECG signals, as the case may be for the certain ECG electrode.

In preferred embodiments, the biasing mechanism can cause the certain ECG electrode to transition from the unbiased state to the biased state, responsive to a monitoring condition being met. As such, the biasing mechanism can be configured to receive a biasing signal that signifies that the determination has been made that the monitoring condition has been met. For example, when a logic device has been provided to make that determination, the biasing mechanism can be configured to receive the biasing signal from that logic device. An example was described above for biasing signal BA.

The transitioning from the biased state to the unbiased state is also called biasing and deployment of the certain ECG electrode. Deployment is for the certain ECG electrode, and possibly also other electrodes of the system. It will be appreciated that deployment in this sense might not necessarily change much the position of the certain ECG electrode with respect to the patient's body, but it will change the force with which it is pushed or biased towards the body.

The biasing mechanism can be made in any way so as to cause pressure to be applied to the certain ECG electrode against the support structure, and therefore bias the certain ECG electrode towards the patient's body. Various embodiments of the biasing mechanism include a spring that is released, causing the support structure to be tightened around the body, causing a balloon to be inflated, adding pressure to a hydraulic system, applying force such as with an electromagnet, turning a screw gun arrangement so that turning result in a translation motion, and or tightening a belt around the body that makes multiple such ECG electrodes come in better contact with the body.

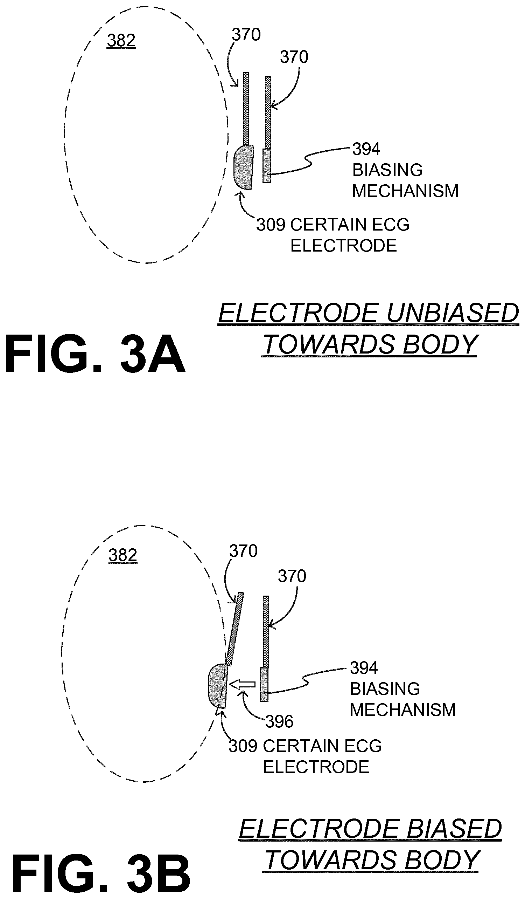

A sample deployment is now described. FIG. 3A is a diagram according to an embodiment. A patient 382 is wearing a support structure 370, of which two portions are shown. Support structure 370 is made according to embodiments and, as with FIG. 1, two parts of it are indicated only generically. A certain ECG electrode 309 is coupled to support structure 370 in an unbiased state. In the instant of FIG. 3A, certain ECG electrode 309 does not even contact the skin of patient 382. A biasing mechanism 394 is also coupled to support structure 370.

FIG. 3B is a diagram showing the same elements as FIG. 3A, except that biasing mechanism 394 exerts a biasing force 396 due to which certain ECG electrode 309 is biased towards patient 382 against support structure 370. In the instant of FIG. 3B, certain ECG electrode 309 contacts the skin of patient 382. Certain ECG electrode 309 is less easily movable in FIG. 3B than in FIG. 3A.

In a number of embodiments the biasing mechanism is preferably made so that it is further reversible, either by the wearer, or by a bystander, or by a remotely monitoring medical professional. Reversing could be upon verifying that there is no actionable episode to be addressed by the system. Reversing could be automatically enabled by further functionality. Or, reversing could be implemented by permitting the mechanically reverse motion of what deployed the certain ECG electrode and any other electrodes. Care should be taken that reversing is not suggested prematurely, or by a patient who does not understand the function of the system, such as a well-meaning by uninformed bystander.

It will be appreciated that, when the certain ECG electrode is in the biased state, it can be counted on to make better electrical contact with the body. Accordingly, any ECG inputs received by the certain ECG electrode preferably are trusted more when the certain ECG electrode is in the biased state than in the unbiased state. Regardless, in a preferred embodiment, an additional, serendipitous check on the patient can be an ECG reading that is received incidentally while the certain ECG electrode is in the unbiased state and whose content causes alarm. Such an ECG reading can be used in a number of ways.

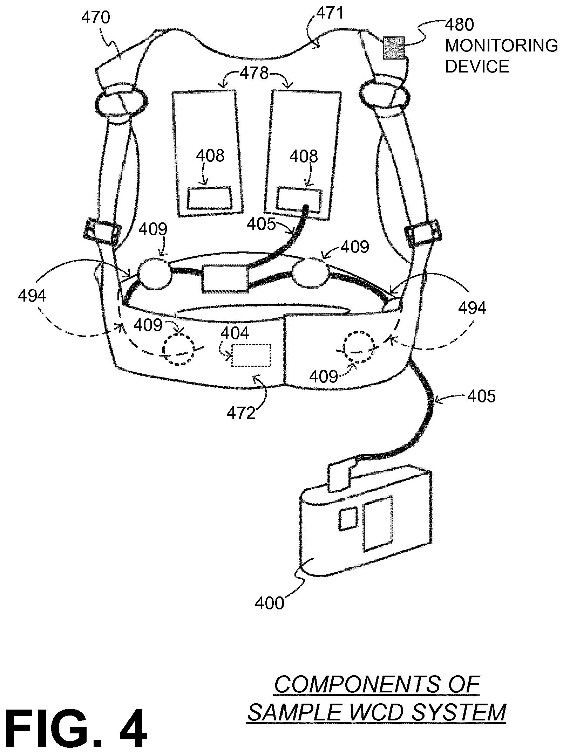

FIG. 4 is a diagram of sample embodiments of components of an WCD system. A support structure 470 includes a flexible vest-like wearable garment. Support structure 470 has a back side 471, and a front side 472 that closes in front of the chest of the patient.

The WCD system of FIG. 4 also includes an external defibrillator 400. FIG. 4 does not show any support for external defibrillator 400, which may be carried in a purse, on a belt, by a strap over the shoulder, and so on. Wires 405 connect external defibrillator 400 to electrodes 404, 408, 409. Of those, electrodes 404, 408 are defibrillation electrodes, and electrodes 409 are ECG sensing electrodes.

Support structure 470 is configured to be worn by the ambulatory patient so as to maintain electrodes 404, 408, 409 on a body of the patient. Indeed, back defibrillation electrodes 408 are maintained in pockets 478. Of course, the inside of pockets 478 can be made with loose netting, so that electrodes 408 can contact the back of the patient, especially with the help of the conductive fluid that has been deployed. In addition, sensing electrodes 409 are maintained in positions that surround the patient's torso, for sensing ECG signals and/or the impedance of the patient.

Moreover, a biasing mechanism includes a string 494, which is preferably provided near, and in fact behind ECG electrodes 409. String 494 may be provided wholly within vest 470, so as not to interfere with the user. The two ends of it can be attached to the garment, preferably near where the garment secures in the front. A mechanism, not shown, may tighten string 494, which biases ECG electrodes 409 inwards towards the body of the patient.

Furthermore, a monitoring device 480 may be provided for the patient, in connection with the system. Such a monitoring device 480 can be as described for the non-ECG parameters of the patient described in connection with devices 180, 281.

ECG signals in a WCD system may include too much electrical noise to be useful. To ameliorate the problem, multiple ECG electrodes 409 are provided, for presenting many ECG sensing options to processor 230. These options are different vectors for sensing the ECG signal, as described now in more detail.

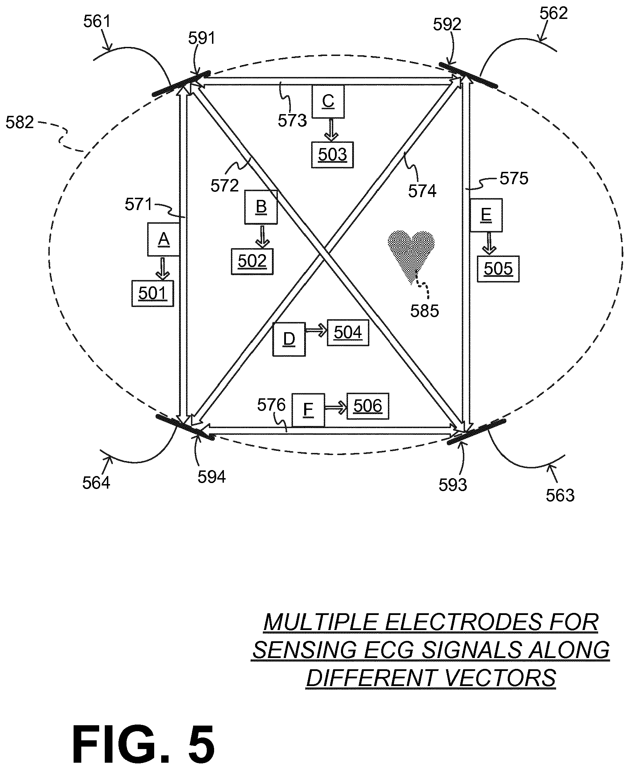

FIG. 5 is a conceptual diagram for illustrating how multiple electrodes of a WCD system may be used for sensing ECG signals along different vectors according to embodiments. A section of a patient 582 having a heart 585 is shown. In FIG. 5, patient 582 is viewed from the top, patient 582 is facing downwards, and the plane of FIG. 5 intersects patient 582 at the torso of the patient.

Four ECG sensing electrodes 591, 592, 593, 594 are maintained on the torso of patient 582, and have respective wire leads 561, 562, 563, 564. It will be recognized that electrodes 591, 592, 593, 594 surround the torso, similarly with ECG electrodes 409 in the example of FIG. 4.

Any pair of these four ECG sensing electrodes 591, 592, 593, 594 defines a vector, along which an ECG signal may be sensed and/or measured. As such, electrodes 591, 592, 593, 594 define six vectors 571, 572, 573, 574, 575, 576. FIG. 5 thus illustrates a multi-vector embodiment.

These vectors 571, 572, 573, 574, 575, 576 define channels A, B, C, D, E, F respectively. Concurrent ECG signals 501, 502, 503, 504, 505, 506 may thus be sensed and/or measured from channels A, B, C, D, E, F, respectively, and in particular from the appropriate pairings of wire leads 561, 562, 563, 564 for each channel.

In FIG. 5 it will be understood that ECG electrodes 591, 592, 593, 594 are drawn as being on the same plane for simplicity and as is preferred, while that is not necessarily the case. Accordingly, vectors 571, 572, 573, 574, 575, 576 are not necessarily on the same plane, either.

In embodiments, in order to make the shock/no-shock determination as correctly as possible, a WCD may assess which of ECG signals 501, 502, 503, 504, 505, 506 is best for rhythm analysis and interpretation. For example, ECG signals that have the most noise may be ignored, discarded, not considered, while leaving the remaining ECG signals as candidates for making the shock/no shock determination.

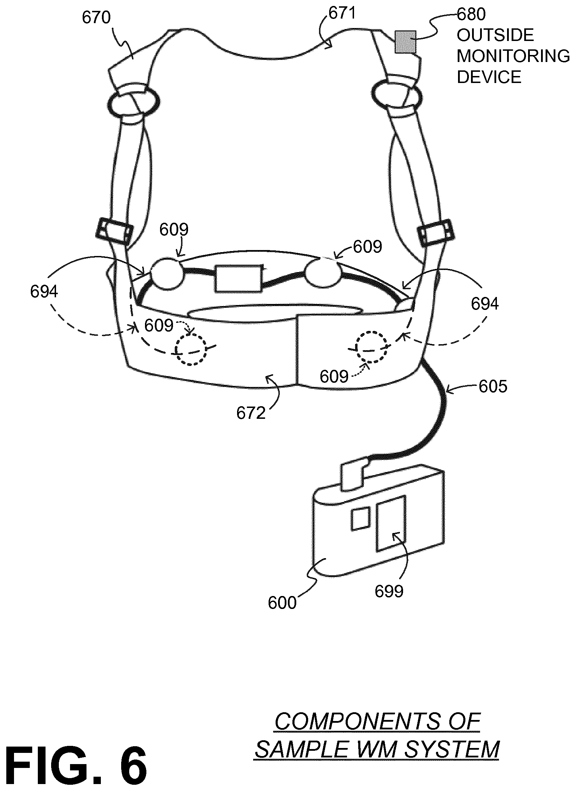

In some embodiments, a wearable medical (WM) system (WMS) has aspects similar to those of a WCD system, except it does not include the defibrillator and the defibrillator electrodes. For example, FIG. 6 is a diagram of sample embodiments of components of an WM system. Of course, a WCD system according to embodiments is also a type of a wearable medical monitor system.

In FIG. 6, a support structure 670 includes a vest-like wearable garment. Support structure 670 has a back side 671, and a front side 672 that closes in front of the chest of the patient.

The WM system of FIG. 6 also includes a device 600. FIG. 6 does not show any support for device 600, which may be carried in a purse, on a belt, by a strap over the shoulder, and so on, or on support structure 670. Wires 605 connect device 600 to ECG electrodes 609.

Support structure 670 is configured to be worn by the ambulatory patient so as to maintain ECG sensing electrodes 609 on a body of the patient. In addition, ECG sensing electrodes 609 are maintained in positions that surround the patient's torso. The ECG electrodes thus define two or more channels and are configured to sense two or more versions of an ECG signal of the patient across the two or more channels. Moreover, a biasing mechanism includes a string 694, similarly with string 494.

Device 600 may have a WM processor and a memory such as was described for processor 230 and memory 238. As such, device 600 could also be storing WM system data that is generated from samples taken during at least one hour of the sensed ECG signal. This stored WM system data can be about patient 82, according to embodiments.

Device 600 may also have a screen 699, which can be an output device of a user interface (UI). Screen 699 can also be an input device of the UI if it is a touchscreen. In some embodiments, all the processing is done by the WM processor, and the results and graphs are displayed in screen 699.

Furthermore, a monitoring device 680 may be provided for the patient, in connection with the system. Such a monitoring device 680 can be as described for the non-ECG parameters of the patient described in connection with devices 180, 281.

A number of embodiments monitor a patient for deterioration of their ECG signal, which could be due to an ailment developing over some time. In such embodiments, a memory of the WM system may store a reference template that is made from one or more early portions of the ECG signal of the ambulatory patient that are sensed during a first time duration. Later, while the patient is monitored, a test portion of the ECG signal may be input, and compared against the reference template. In some of these embodiments, the WM system is actually a wearable cardioverter defibrillator (WCD) system, which also includes one or more defibrillation electrodes that the support structure further maintains on the patient's body. Such a WCD system also includes an energy storage module that store an electrical charge, which is discharged via the defibrillation electrodes through the patient. Some of these embodiments are now described.

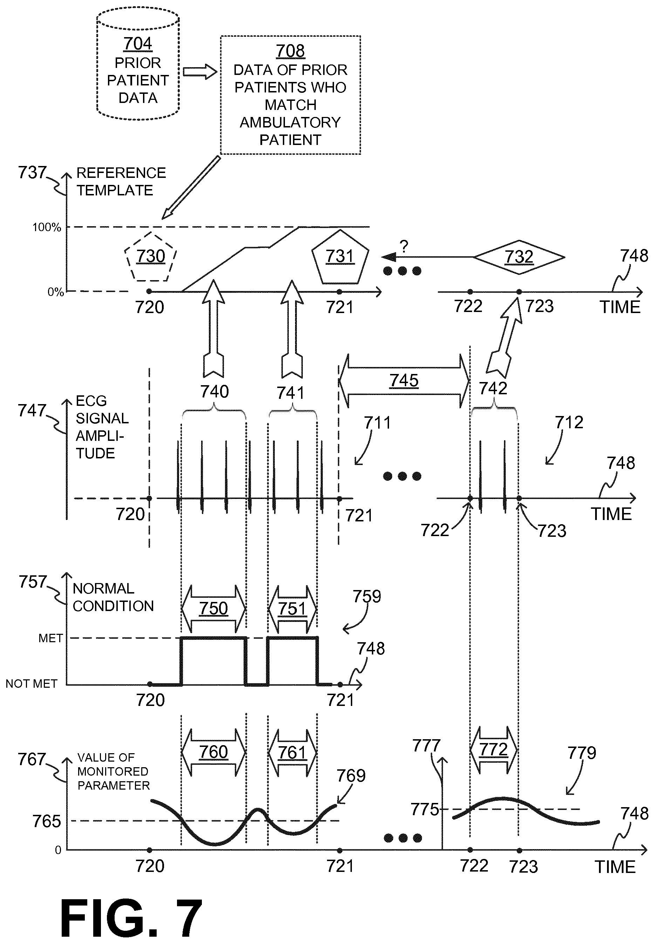

FIG. 7 shows multiple diagrams and relationships among them. In about the middle of the diagram, against a vertical axis 747, the ECG signal amplitude is plotted over a time axis 748 that is discontinued in one place. Two portions of the ECG signal amplitude are plotted, namely an early portion 711 and a later portion 712.

Early portion 711 is sensed within a first time duration. The first time duration is defined, in this example, as occurring between time intercepts 720 and 721. Later portion 712 is sensed within a second time duration. The second time duration is defined as occurring between time intercepts 722 and 723. The second time duration occurs after the first time duration. In fact, the second time duration can start at least 10 min or longer after the first time duration ends. In other words, the duration between time intercepts 721 and 722, which is also shown by an arrow 745, is at least 10 min. This duration can be minutes, hours, days, weeks and months, as the patient is monitored for the long term, and much longer than what happens during the first or second time duration. This is why time axis 748 is shown discontinued for the time duration of arrow 745.

In this example, the ECG signal of early portion 711 is used to make a reference template 731. The state of completion of reference template 731 is shown conceptually against a vertical axis 737, over time axis 748 that is repeated from the diagram immediately below. In this diagram the completion is shown as rising gradually from 0% to 100%, but that is for illustrating the concept only. In embodiments, and as will be seen below the rise is not gradual. When there are enough number of normal QRS beats in a time interval, then reference template 731 can be formulated. It will be appreciated that, when the first time duration is complete at time intercept 721, reference template 731 is complete, having reached a state of completion of 100%. Then, at time intercept 723, a comparison operation 732 may be made. The comparison may be of a test portion 742 of ECG signal 712 that is sensed during second time duration 742 against reference template 731. Examples of such a comparison are given later in this document.

Returning to reference template 731, it will be appreciated that it was created with portions 740, 741 of ECG signal 711, during the first time duration. However, a system according to embodiments might not always start with a reference template 731, which has been made from the patient's data. In some embodiments, a default template 730 is optionally stored in the memory of the WM system before reference template 731 is made or completed. In such embodiments, default template 730 can be used instead of reference template 731. In particular, comparison operation 732 may determine a difference by comparing the value of the test aspect of the inputted test portion 712 of the ECG signal with a value of the reference aspect of default template 730 instead of with the value of the reference aspect of reference template 731.

In such embodiments, default template 730 may have been constructed from prior data of prior patients, such as may be in a database 704. In some of these embodiments, prior data 708 is selected from database 704, to be from prior patients who match the ambulatory patient in at least one demographic parameter of a set that includes: gender, age, height, weight, and so on. It should be noted that ECG data can be different in some aspects if they are sourced from a standard "12-lead" measurement, than if they are sourced from ECG electrodes of the type shown in FIG.s 4-6.

Making or constructing reference template 731 is now described in more detail.

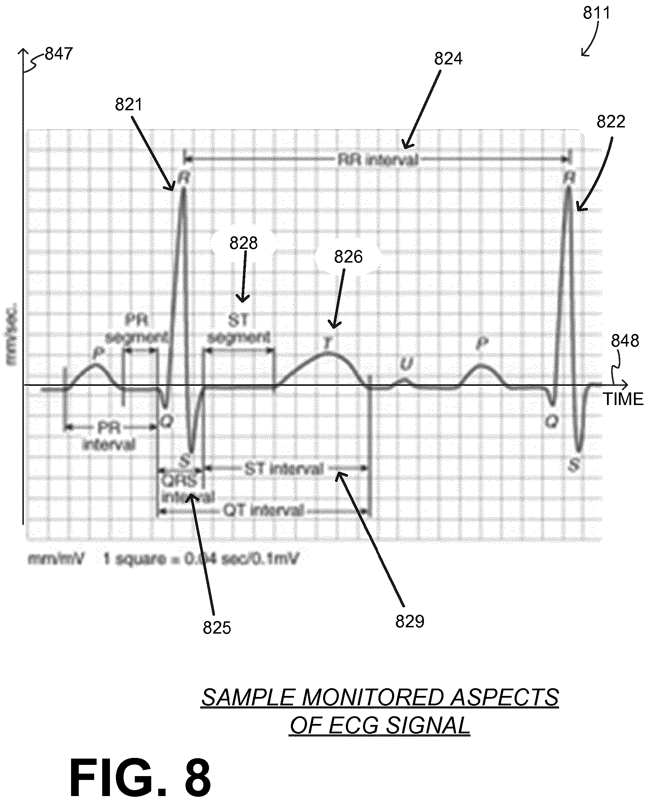

In some embodiments, the processor of the WM system is further configured to select an amplitude waveform of a certain one of the one or more early portions 711 of the ECG signal, and store the selected amplitude waveform in the memory as the reference template. For example, FIG. 8 is a time diagram 811 of a segment of a waveform amplitude of an ideal ECG signal, assuming the patient had one that was captured this way. A non-ideal, and more usual, ECG signal amplitude waveform 1211 is also shown in a later drawing, but for a different purpose. Even if the patient's signal is not ideal, FIG. 8 serves well to indicate aspects that can be monitored according to embodiments of operation 732. The ECG amplitude waveform is shown against a vertical axis 847, and over a time axis 848. Two QRS peaks 821, 822 are included. Aspects that can be monitored according to embodiments, such as test aspects and reference aspects, include an RR interval 824, a QRS interval 825, a T-wave duration 826, an ST segment duration 828 and an ST interval duration 829. These aspects can have values given, for example, by their intercepts on axes 847, 848. In each case, the portion of the waveform stored as the reference template is chosen according to the aspect that it is desired to monitor. Of course, more than one aspects may be thus monitored according to embodiments, and so on.

In other embodiments, reference template 731 is made or constructed using multiple waveforms, in which case it can also be called a composite amplitude waveform. In particular, the processor of the WM system can be further configured to generate such a composite amplitude waveform from early amplitude waveforms of respective ones of early portions 740, 741 of the ECG signal. The composite amplitude waveform can be generated by any number of techniques, such as adding, averaging, choosing by median, a combination of such techniques, and so on. The processor can then store the composite amplitude waveform in the memory as reference template 730. Again, if multiple aspects of the signal are to be thus monitored, then reference template 730 may have multiple aspects, each with one or more values, and so on.

In some of these embodiments, the composite amplitude waveform can be generated from early amplitude waveforms that are similar to each other. The similarity can be at least in the aspects mentioned above in FIG. 8, and also in other aspects. In fact, the similarity can be quantified as internal correlation, to indicate how well-correlated are these amplitude waveforms. For example, a processor of the WM system can be further configured to identify similarity statistics of early amplitude waveforms of respective ones of the early portions of the ECG signal. Then the composite amplitude waveform can be generated from those of the early amplitude waveforms whose similarity statistics exceed a threshold.

In some of these embodiments, the processor is further configured to align the early amplitude waveforms, before generating the composite amplitude waveform from them. Aligning can help with the resulting composite amplitude waveform being a better answer. Filtering can be performed in a number of ways. One such example is by a bandpass filter. Such a filter may pass frequencies from 8 Hz to 25 Hz.

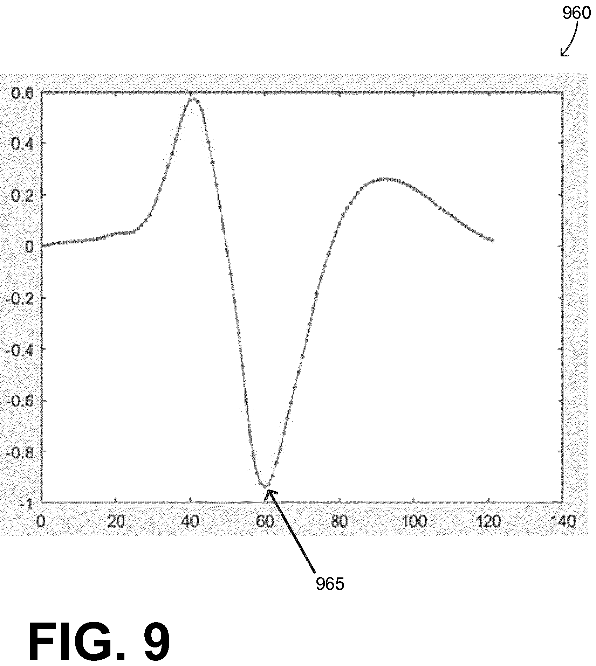

In some of these embodiments, the processor is further configured to filter the early amplitude waveforms prior to aligning them. Filtering can help with the alignment, as it may sharpen a peak. An example is seen in FIG. 9, which shows a time diagram 960 of a sample ECG waveform that has been filtered. Alignment can be by aligning the early amplitude waveforms by their sharp peak 965.

The devices and/or systems mentioned in this document may perform functions, processes, acts, operations, actions and/or methods. These functions, processes, acts, operations, actions and/or methods may be implemented by one or more devices that include logic circuitry. A single such device can be alternately called a computer, and so on. It may be a standalone device or computer, such as a general-purpose computer, or part of a device that has and/or can perform one or more additional functions. The logic circuitry may include a processor and non-transitory computer-readable storage media, such as memories, of the type described elsewhere in this document. Often, for the sake of convenience only, it is preferred to implement and describe a program as various interconnected distinct software modules or features. These, along with data are individually and also collectively known as software. In some instances, software is combined with hardware, in a mix called firmware.

Moreover, methods and algorithms are described below. These methods and algorithms are not necessarily inherently associated with any particular logic device or other apparatus. Rather, they are advantageously implemented by programs for use by a computing machine, such as a general-purpose computer, a special purpose computer, a microprocessor, a processor such as described elsewhere in this document, and so on.

This detailed description may include flowcharts, display images, algorithms, and symbolic representations of program operations within at least one computer readable medium. An economy may be achieved in that a single set of flowcharts can be used to describe both programs, and also methods. So, while flowcharts describe methods in terms of boxes, they may also concurrently describe programs.

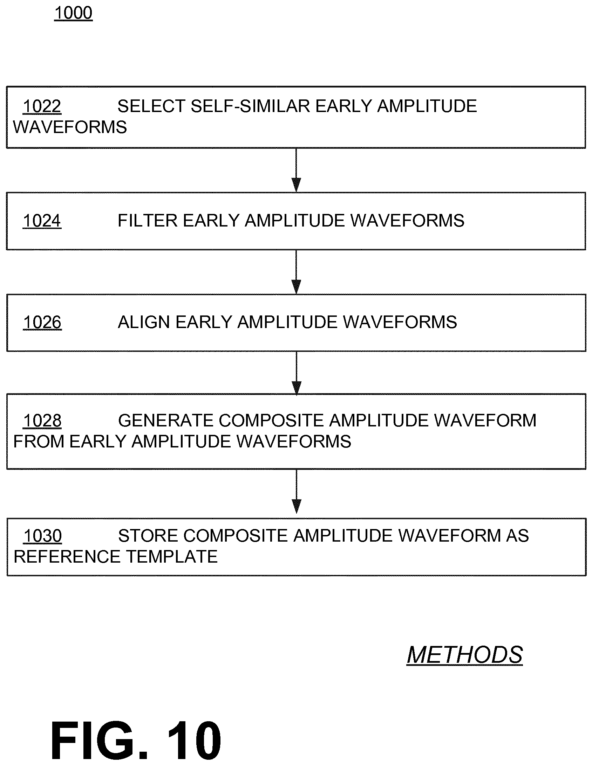

FIG. 10 shows a flowchart 1000 for describing methods according to embodiments. According to an optional operation 1022, self-similar early waveforms may be selected. These early waveforms can be amplitude waveforms. The selection may be performed, for example, by identifying similarity statistics of early amplitude waveforms of respective ones of the early portions of the ECG signal, and selecting those of the early amplitude waveforms whose similarity statistics exceed a threshold.

According to another, optional operation 1024, early amplitude waveforms may be filtered.

According to another, optional operation 1026, the early amplitude waveforms may be aligned, meaning shifted along the time axis. Shifting can be so that one of their key features matches, such as sharp peak 965.

According to another, optional operation 1028, a composite amplitude waveform may be generated from the early amplitude waveforms.

According to another, optional operation 1030, the composite amplitude waveform may be stored in the memory as the reference template.

Returning to FIG. 7, it will be appreciated that, in this example, reference template 731 was completed with contributions of only ECG signal portions 740, 741, and not from other portions of the ECG signal of the first time duration. This refinement is not required, but is preferred, to favor using ECG segments during condition that are regarded as normal. For example, in some embodiments, a processor of the WM system is further configured to determine whether or not a normal condition was met when a certain one of the early portions of the ECG signal was sensed. In such embodiments, the early amplitude waveform of the certain early portion is used to generate the composite amplitude waveform, responsive to determining that the certain early portion was sensed when a normal condition was met. If, however, the normal condition was not met, that early amplitude waveform might not be used to generate the composite amplitude waveform.

An example is seen in FIG. 7, where a vertical axis 757 is used to denote two values, namely normal condition met and not met. In this example, a plot 759 plots the evolution of that value over time axis 748. Plot 759 indicates that the normal condition is met only during time intervals 750 and 751. It will be appreciated that these time intervals 750 and 751 are what define ECG portions 740 and 741 of the diagram immediately above.

The normal condition can be defined in a number of ways. For one example, the normal condition may include that a heart rate of the patient is less than a heart rate threshold. A good heart rate threshold may be 80 bpm (beats per minute). For another example, the normal condition may include that QRS complexes appear normally, and so on. Moreover, it may be desirable to set the normal condition when the patient is sleeping, or in a supine position but not sleeping, or sitting, or standing still, which can be confirmed with a variety of sensors, such as sound sensors, motion sensors, a clock input and so on.

In some such embodiments, the WM system may further include a monitoring device configured to monitor at least one physiological parameter of the ambulatory patient that is not the ECG signal of the ambulatory patient. In such embodiments, the normal condition is met responsive to a value of the monitored parameter being less than a normal threshold. An example is seen in FIG. 7 by a plot 769. The value of such a monitored parameter is tracked against a vertical axis 767 over time axis 748. Plot 769 indicates that the normal condition is met only during time intervals 760 and 761, because that is when a normal threshold 765 is not exceeded. It will be appreciated that these time intervals 760 and 761 are what created plot 759 immediately above, and therefore defined ECG portions 740 and 741 of the diagram further above.

The physiological parameter that is monitored can be any one of the patient's heart rate, blood perfusion, blood flow, blood pressure, blood oxygen level, pulsatile change in light transmission or reflection properties of perfused tissue, heart sounds, breathing sounds, pulse, and motion. Accordingly, a monitoring device of the WM system can include a sensor, for detecting when the condition is normal. The sensor can be appropriate for sensing and producing a value for the physiological parameter that is being monitored.

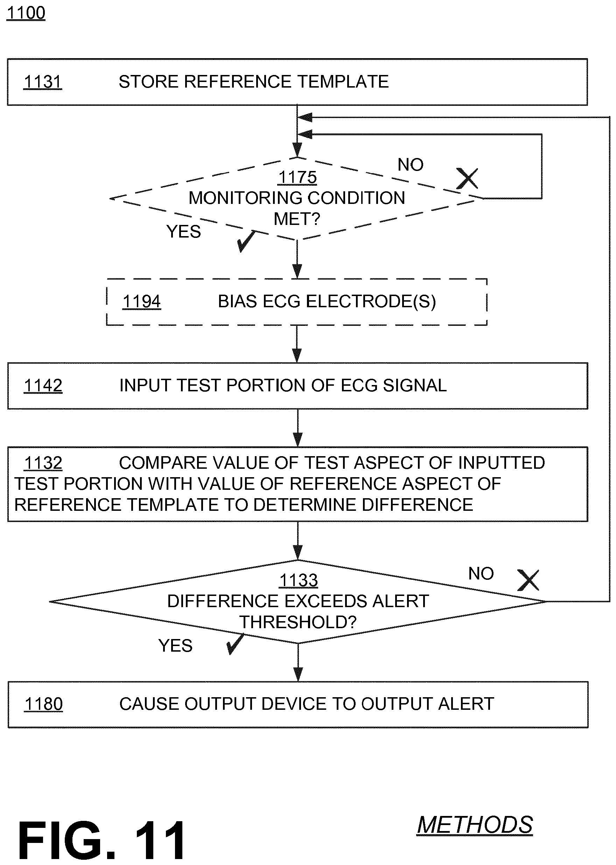

FIG. 11 shows a flowchart 1100 for describing methods according to embodiments. According to an operation 1131, a reference template may be stored in a memory of the WM system.

According to another, optional operation 1175, it may be determined whether or not a monitoring condition is met. Examples are given elsewhere in this document. If not, then operation 1175 may be performed again, perhaps after some delay.

If yes then, according to another, optional operation 1194, ECG electrodes may be biased towards the patient. This can be performed in a number of ways. For example, a certain one of the ECG electrodes may be caused, by biasing mechanism 194, 394, to transition from the unbiased state to the biased state. In addition, this biasing may also take place for the first time period, when the normal condition is met. After being performed, the biasing may be later reversed to relieve the patient, and so on.

According to another operation 1142, a test portion of the ECG signal may be input. Such a test portion maybe sensed during a second time duration that starts at least 10 min after the first time duration ends. Inputting may be by sensing from the patient, or by recalling a signal stored in the memory.

According to another operation 1132, a value of a test aspect of the inputted test portion may be compared with a value of a reference aspect of the reference template stored in the memory, to determine a difference. This is similar to operation 732. In addition, waveforms of the test portion of the ECG signal may be aggregated for the comparison. As such, the test aspect may include an aggregated waveform of the test portion of the ECG signal.

According to another operation 1133, it is determined whether the difference determined at operation 1132 exceeds an alert threshold. If not, then execution may return to an earlier operation such as operation 1175.

If yes then, according to another operation 1180, an output user interface device can be caused to output an alert.

Monitoring conditions, such as of operation 1175, can be similar to what was described for determining whether or not a normal condition is met. For example, the WM system may further include one or more monitoring devices, such as those described in connection with determining whether or not a normal condition is met. These monitoring devices can now determine instead whether or not a monitoring condition is met. The monitoring condition is not necessarily the same as the normal condition.

An example is seen in FIG. 7 by a plot 779. The value of such a monitored parameter is tracked against a vertical axis 777 over time axis 748. Plot 779 indicates that the monitoring condition is met only during time interval 772, because that is when plot 779 exceeds monitoring threshold 775. It will be appreciated that this time interval 772 is what defined ECG portion 742 of the diagram above.

It will be further observed that monitoring threshold 775 does not necessarily have the same value as normal threshold 765. In case where they are both heart rate thresholds, monitoring threshold 775 could be set at a suitable value, such as 90 bpm.

Another example is when the processor further includes a clock configured to keep a time. The monitoring condition can be met responsive to the time reaching a checking moment. One more example is when the WM system further includes a user interface (UI) having an input device that is configured to be actuated by the patient. The monitoring condition may be met responsive to the patient actuating the input device on his own.

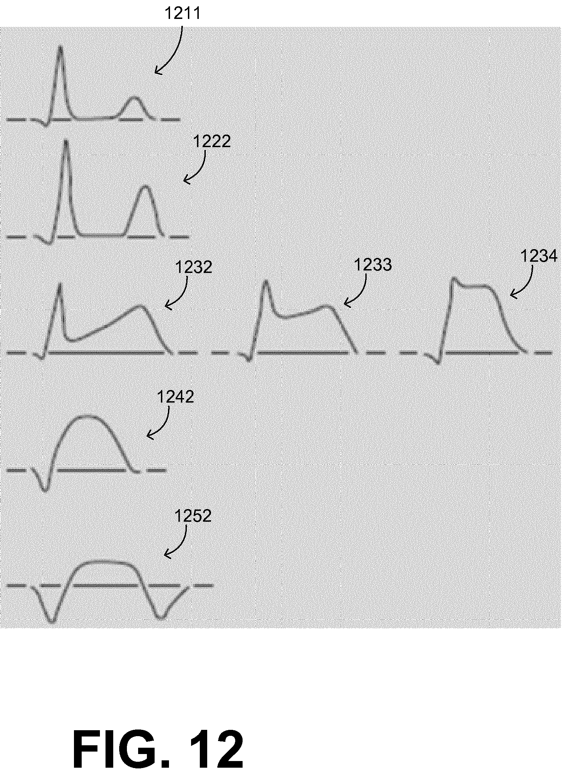

FIG. 12 shows sample time diagrams of a healthy and of suspect ECG waveforms that can be detected by embodiments. Waveform 1211 is deemed as a relatively healthy one, where a QRS peak is followed by a T-wave. Waveform 1211 is presented also for easier contrast with other waveforms in this diagram, which are not as healthy. Indeed, waveform 1222 exhibits a peaked T-wave, while waveforms 1232, 1233, 1234 exhibit increasing degrees of ST segment elevation. Waveform 1242 exhibits Q wave formation and loss of R wave, while waveform 1252 exhibits T-wave inversion.

Unhealthy waveforms, such as those of FIG. 12, may be detected by a suitable choice of the test aspect. For example, the test aspect may include a QRS interval of a QRS complex of the inputted test portion. Or, the test aspect may include an ST segment or an ST interval after a QRS complex of the inputted test portion. Or, the test aspect may include a T-wave after a QRS complex of the inputted test portion. In these instances, the choice of the test aspect indicates the corresponding choice of the reference aspect. Detection can be facilitated by aligning waveforms as mentioned above, and then examining a difference in other aspects.

In some embodiments, the difference of operation 1132 is determined by computing a waveform similarity coefficient. In such embodiments, the alert can be output responsive to the computed waveform similarity coefficient being below a similarity threshold.

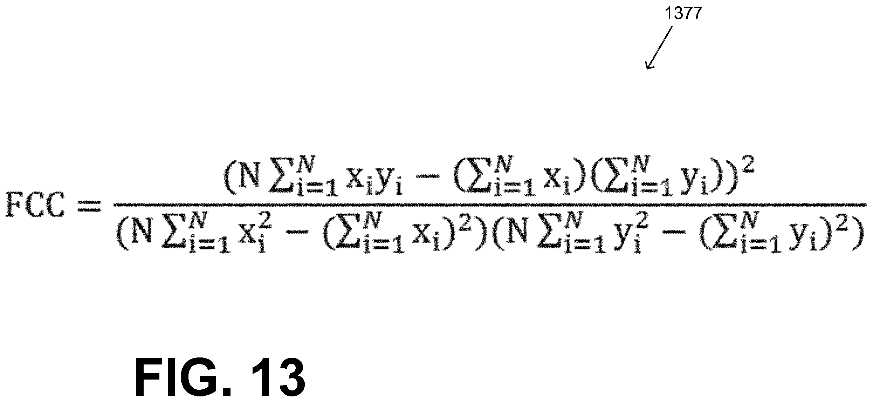

In some of these embodiments, the waveform similarity coefficient is computed according to equation 1377 in FIG. 13 of this document. In equation 1377, FCC stands for feature correlation coefficient. Its value ranges from 1, which amounts to perfect correlation, to 0, which means complete lack of correlation. As an aside, such an FCC statistic can also be used to measure the stability and self-similarity of the early waveforms that constitute the reference template. A good value for N can be chosen depending on the sampling rate and the QRS-T duration. In some embodiments, a value of about 8 points works well. Examples of using the FCC are now described.

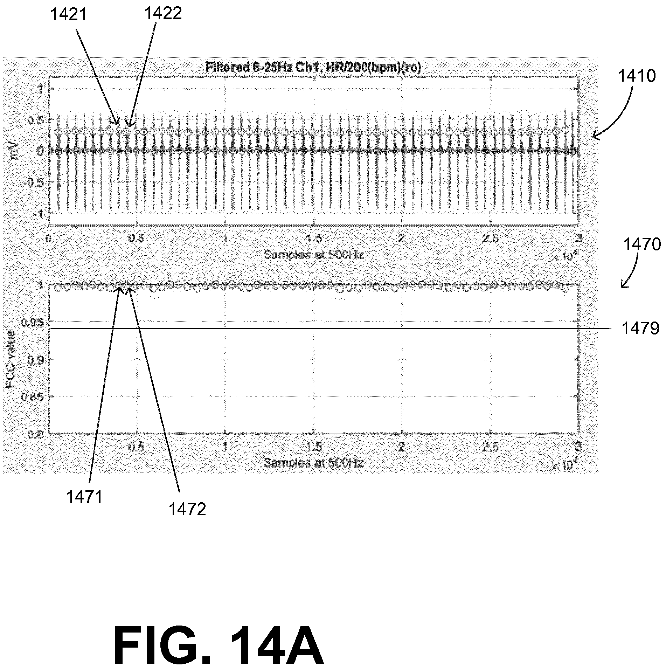

FIG. 14A shows a time diagram of an ECG signal 1410, with QRS peaks that appear to the human eye as parallel vertical lines. Little circles 1421, 1422 indicate where QRS peaks have been detected. ECG signal 1410 is taken during a patient's regular heartbeat.

FIG. 14A also shows another time diagram 1470, on the same time axis as ECG signal 1410. Second time diagram 1470 plots the waveform similarity coefficients for the peaks detected in the above diagram, which have been computed according to equation 1377 in FIG. 13. Of those, two sample coefficients 1471, 1472 are shown, which correspond to QRS peaks 1421, 1422 respectively. It will be appreciated that, in this example, all coefficients are above a threshold line 1479, which corresponds to a value of 0.94. In fact, all are much higher, all much closer to 1 and all rather similar to each other. As such, ECG signal 1410 is rather well-correlated. A metric can be established suitably for quantifying this internal correlation.

The waveforms of ECG signal 1410 may be aggregated, for example by extracting an average. Given the sampling rate of 500 Hz, the shown 30,000 samples of ECG signal 1410 would give about 60 sec of signal, which is adequate for aggregating. For averaging, prior to adding the waveforms are preferably aligned, and prior to aligning they are first filtered, as described above at least in FIG. 10.

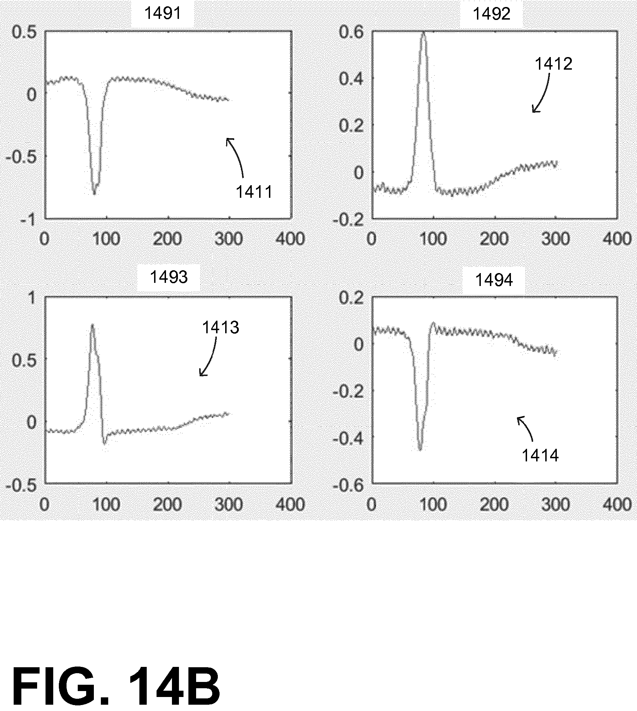

The aggregated waveforms can form an average QRS_T waveform of each unfiltered ECG signal. The results are shown in FIG. 14B, which shows sample concurrent aggregated signals 1411, 1412, 1413, 1414, after removing the DC offset. These aggregated signals correspond to ECG electrodes 1491, 1492, 1493, 1494, and are with respect to common reference voltage, and are thus also called single-ended. ECG electrodes 1491, 1492, 1493, 1494 could be, for example ECG electrodes 591, 592, 593, 594. The signal from the channel, however, would be by taking a difference of these signals. Aggregated signals 1411, 1412, 1413, 1414 can also be used as reference templates, one for the signal of each electrode, or the difference of two per channel. If a prior reference template had been stored but was not well-correlated internally, then signals 1411, 1412, 1413, 1414 can serve as an updated reference template, which is better correlated. In other words, a previous template may have been stored in the memory, before the new reference template is made from aggregated signals 1411, 1412, 1413, 1414. The previous template may have a first metric of internal correlation, but the new reference template may have a second metric of internal correlation. The previous template may be replaced thus by the new reference template responsive to the second metric exceeding the first metric.

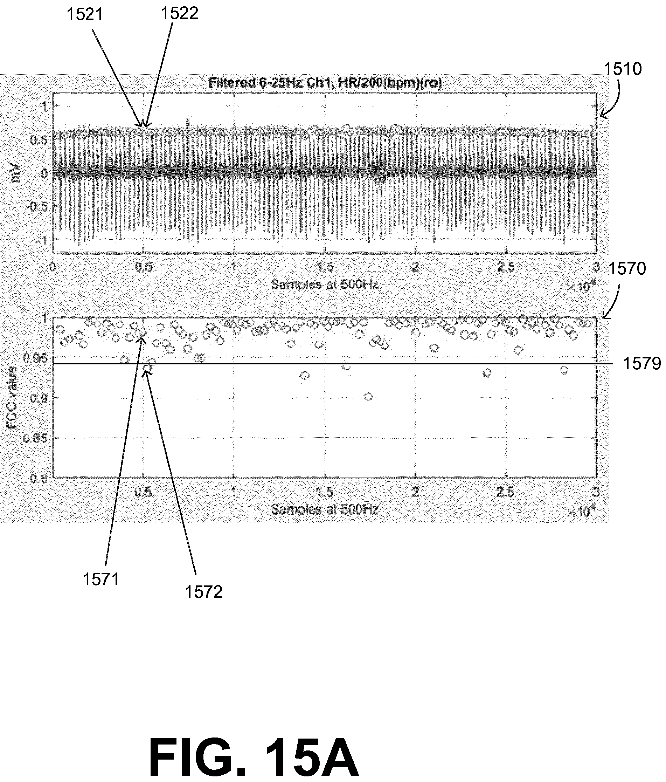

FIG. 15A shows a time diagram of an ECG signal 1510, with QRS peaks that appear to the human eye as parallel vertical lines. Little circles 1521, 1522 indicate where QRS peaks have been detected. ECG signal 1510 is taken during a patient's elevated heart rate of around 120 bpm.

FIG. 15A also shows a time diagram 1570, on the same time axis as ECG signal 1510. Second time diagram 1570 plots the waveform similarity coefficients for the peaks detected in the above diagram, which have been computed according to equation 1377 in FIG. 13. Of those, two sample coefficients 1571, 1572 are shown, which correspond to QRS peaks 1521, 1522 respectively. In this example, coefficient 1572 is not above a threshold line 1579, which corresponds to a value of 0.94. As such, ECG signal 1510 is less well-correlated internally than ECG signal 1410.

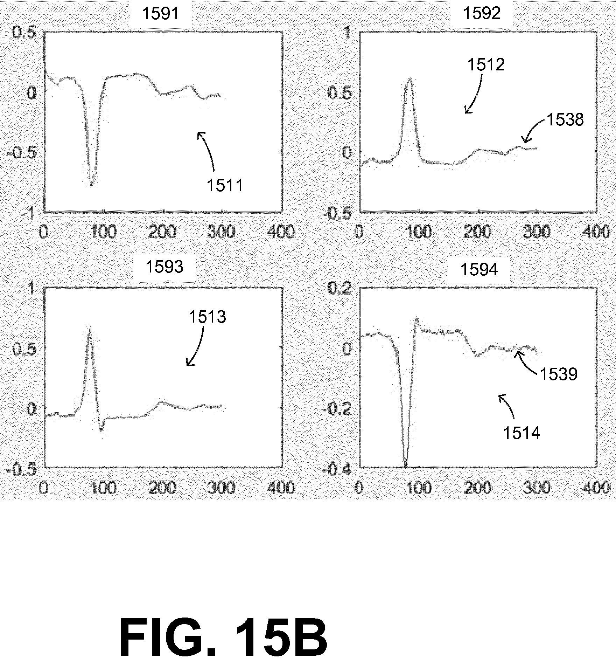

The waveforms of ECG signal 1510 may be aggregated, for example by extracting an average, as with FIG.s 14A, 14B. The results are shown in FIG. 15B, which shows sample concurrent aggregated signals 1511, 1512, 1513, 1514, after removing the DC offset. These aggregated signals correspond to ECG electrodes 1591, 1592, 1593, 1594, and are with respect to common reference voltage. ECG electrodes 1591, 1592, 1593, 1594 could be, for example ECG electrodes 591, 592, 593, 594, or even 1491, 1492, 1493, 1494 but at a later time than in FIG. 14A.

If aggregated signals 1411, 1412, 1413, 1414 had become stored as the reference templates, and aggregated signals 1511, 1512, 1513, 1514 are compared to them as later test portions, the FCC may have been used for computing the difference for operation 1132. In such a case, ECG signal 1510 is a test portion of the ECG signal, and aggregated signals 1512, 1514 are test aspects of it. The difference for operation 1132 includes new features 1538, 1539. They correspond to a T-wave variation relative to the reference templates, which can happen when the heart is stressed. Such stress to the heart may happen during an activity such as running, shoveling snow, etc. The ST segment can change, by being elevated or depressed, which can be a heart attack symptom. As such, the patient and/or a clinician may be notified, and the patient may stop the activity, if that were the problem. Of course, ST segment changes can occur during a myocardial infarction/heart attack independently of the patient's activity. Plus, activity-induced ST segment changes can be indicative of an issue other than heart attack, such as such as coronary spasm.

In some embodiments, the processor is further configured to store in the memory an event responsive to the difference exceeding the alert threshold. This way, the event may be reviewed later.

The alert of operation 1180 may be output in a number of ways. In some embodiments, the WM system includes a user interface (UI), and the output device belongs to the UI. In such embodiments, the alert is output by the output device emitting a human-perceptible indication, such as a sound, a light, a vibration, and so on.

In other embodiments, the WM system includes a communication module that is the output device. In such embodiments, the alert is output by the communication module transmitting a notification to another device, such as a computer, a telephone, and so on, for example for a remote person to react.

In some embodiments, the memory of the WM system further stores a table that lists a plurality of possible ailments of the ambulatory patient. Such ailments may include a higher heart rate, a caution about a possible heart attack, decompensation, stroke, and heart failure or the possibility of the onset of heart failure. For example, reference templates may be stored for such ailments, comparisons can be made for matching such ailments, and so on.

A number of embodiments monitor a patient for a myocardial infarction (MI), such as an acute MI (AMI), by monitoring primarily a physiological parameter of the patient that is not the ECG signal of the patient. In such embodiments, a memory of the WM system may store one or more myocardial infarction (MI) alarm conditions that are related to an MI. While the patient is monitored, a value of the monitored physiological parameter is input, and it is determined whether or not that value meets at least one of the one or more MI alarm conditions. In some of these embodiments, the system is also a wearable cardioverter defibrillator (WCD) system, which also includes one or more defibrillation electrodes that the support structure further maintains on the patient's body. Such a WCD system also includes an energy storage module that store an electrical charge, which is discharged via the defibrillation electrodes through the patient. Some of these embodiments are now described.

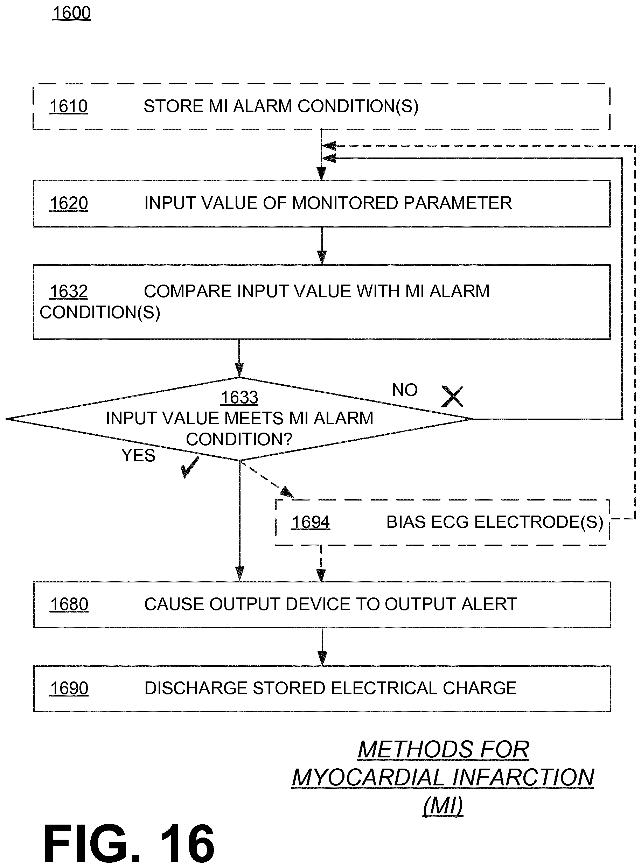

FIG. 16 shows a flowchart 1600 for describing methods according to embodiments.

According to an optional operation 1610, myocardial infarction (MI) alarm conditions are stored, for example in a memory of the WM system. These MI alarm conditions may be related to a myocardial infarction, and further be chosen according to the physiological parameter that is monitored. These MI alarm conditions can be generated as described later in this document.

According to another operation 1620, a value of a monitored physiological parameter may be input. This physiological parameter need not be the ECG signal of the patient, and examples are described elsewhere in this document.

According to another operation 1632 the input value may be compared with the stored MI alarm conditions.

According to another operation 1633, it may be determined, as a result of operation 1632, whether or not the input value meets at least one of the stored one or more MI alarm conditions. If not, then execution may exit, or return to an earlier operation such as operation 1620.

If at operation 1633 the answer is yes, there are two options, depending on whether or not optional operation 1694 is provided for, and/or performed. If not optional operation 1694 is not performed or provided for then, according to another operation 1680, an output device is caused to output an alert. This may be performed, for example, similarly with operation 1180 of FIG. 11. According to another, optional operation 1690, a stored electrical charge may be discharged via a defibrillation electrode through the ambulatory patient.

In some of these embodiments, the WM system further includes electrocardiogram (ECG) electrodes that are configured to be maintained on a body of the ambulatory patient by the support structure. In such embodiments, the ECG electrodes can be configured, when thus maintained, to sense an ECG signal of the ambulatory patient. This ECG signal may provide additional information about a possible MI.

In some of these embodiments, a biasing mechanism is provided, similarly with what was described above. In such embodiments, optional operation 1694 in FIG. 16 can be provided for and performed, responsive to a condition. Such a condition can be any suitable condition of the type described above, such as a monitoring condition and/or determining that the input value meets at least one of the one or more MI alarm conditions at operation 1633. Then ECG electrodes may be biased towards the patient, similarly with operation 1194. Additional operations may then occur similarly with previous embodiments, ultimately leading to operation 1680 or 1620. In particular, a reference template can be further stored in the memory, and a test portion of the ECG signal of the ambulatory patient can be input that is thus sensed by the certain ECG electrode in the biased state. Then a test aspect of the inputted test portion can be compared with a reference aspect of the stored reference template to determine a difference, and the output device can be caused to output an alert responsive to the difference exceeding an alert threshold.

In some of these embodiments, the monitored physiological parameter of the patient includes one or more of heart sounds, a breathing sound, a heart rate, a pulsatile blood flow, a blood oxygen level, a blood perfusion, a change in light transmission or reflection properties of perfused tissue, a color of a skin, and a motion.

For each such monitored physiological parameter, the monitoring device may be made accordingly, with appropriate sensors. A WM system according to embodiments may have one or more types of sensors, and one or more sensors of each type. Outputs of such sensors, and of monitoring devices, may be used individually, or together for higher confidence.

For each type of monitored physiological parameter, the corresponding MI alarm conditions can be generated in relation to the parameter, for example as logical rules for a processor of the WM system. In particular, it can be identified what aspect of the each of the parameters signifies a possible MI.

For example, the monitoring device may include a perfusion detector. Such a perfusion detector may monitor blood perfusion of the patient, and detect perfusion, heart beats, heart beat rate, heart beat intensity, heart beat consistency, etc. The corresponding MI alarm conditions can be generated when these detected quantities are not normal, for example rapid heartbeats (frequency higher than a threshold), irregular heartbeats (a custom statistic about regularity not meeting a threshold), etc.

For another example, the monitoring device may include a microphone. Such a microphone can be configured to monitor the patient's heart sounds and/or breathing sounds, by appropriate placement etc.

For heart sounds, the microphone may detect frequency, volume, intensity, regularity, etc. of heart beats. Again, the corresponding MI alarm conditions can be generated accordingly.

For breathing sounds, the microphone may detect frequency, volume, regularity, etc. of breaths. Again, the corresponding MI alarm conditions can be generated accordingly. For example, a frequency that is too high may indicate shortness of breath. The breathing sounds can also be used for detection of snoring, especially if coupled with confirming indications of conditions related to snoring, such as perhaps ambient light being low, time being that of the night, motion sensors indicating no other motion, and so on.

In the methods described above, each operation can be performed as an affirmative act or operation of doing, or causing to happen, what is written that can take place. Such doing or causing to happen can be by the whole system or device, or just one or more components of it. It will be recognized that the methods and the operations may be implemented in a number of ways, including using systems, devices and implementations described above. In addition, the order of operations is not constrained to what is shown, and different orders may be possible according to different embodiments. Examples of such alternate orderings may include overlapping, interleaved, interrupted, reordered, incremental, preparatory, supplemental, simultaneous, reverse, or other variant orderings, unless context dictates otherwise. Moreover, in certain embodiments, new operations may be added, or individual operations may be modified or deleted. The added operations can be, for example, from what is mentioned while primarily describing a different system, apparatus, device or method.

A person skilled in the art will be able to practice the present invention in view of this description, which is to be taken as a whole. Details have been included to provide a thorough understanding. In other instances, well-known aspects have not been described, in order to not obscure unnecessarily this description.

This description includes one or more examples, but this fact does not limit how the invention may be practiced. Indeed, examples, instances, versions or embodiments of the invention may be practiced according to what is described, or yet differently, and also in conjunction with other present or future technologies. Other such embodiments include combinations and sub-combinations of features described herein, including for example, embodiments that are equivalent to the following: providing or applying a feature in a different order than in a described embodiment; extracting an individual feature from one embodiment and inserting such feature into another embodiment; removing one or more features from an embodiment; or both removing a feature from an embodiment and adding a feature extracted from another embodiment, while providing the features incorporated in such combinations and sub-combinations.

In general, the present disclosure reflects preferred embodiments of the invention. The attentive reader will note, however, that some aspects of the disclosed embodiments extend beyond the scope of the claims. To the respect that the disclosed embodiments indeed extend beyond the scope of the claims, the disclosed embodiments are to be considered supplementary background information and do not constitute definitions of the claimed invention.

Reference numerals: In this description a single reference numeral may be used consistently to denote a single item, aspect, component, or process. Moreover, a further effort may have been made in the preparation of this description to use similar though not identical reference numerals to denote other versions or embodiments of an item, aspect, component or process that are identical or at least similar or related. Where made, such a further effort was not required, but was nevertheless made gratuitously so as to accelerate comprehension by the reader. Even where made in this document, such a further effort might not have been made completely consistently for all of the versions or embodiments that are made possible by this description. Accordingly, the description controls in defining an item, aspect, component or process, rather than its reference numeral. Any similarity in reference numerals may be used to infer a similarity in the text, but not to confuse aspects where the text or other context indicates otherwise.

The claims of this document define certain combinations and subcombinations of elements, features and acts or operations, which are regarded as novel and non-obvious. The claims also include elements, features and acts or operations that are equivalent to what is explicitly mentioned. Additional claims for other such combinations and subcombinations may be presented in this or a related document. The terms used herein, including in the claims, are generally intended as "open" terms. For example, the term "including" should be interpreted as "including but not limited to," the term "having" should be interpreted as "having at least," etc. If a specific number is ascribed to a claim recitation, this number is a minimum but not a maximum unless stated otherwise. For example, where a claim recites "a" component or "an" item, it means that the claim can have one or more of this component or this item.

## Claims

1. A wearable medical (WM) system for an ambulatory patient, comprising:
electrocardiogram (ECG) electrodes;
a support structure configured to be worn by the ambulatory patient so as to maintain the ECG electrodes on a body of the ambulatory patient, the ECG electrodes configured, when thus maintained, to sense an ECG signal of the ambulatory patient;
an output device;
a memory configured to store a reference template, the reference template made from one or more early portions of the ECG signal of the ambulatory patient that are sensed during a first time duration; and
a processor configured to:
input a test portion of the ECG signal of the ambulatory patient that is sensed during a second time duration,
compare a value of a test aspect of the inputted test portion with a value of a reference aspect of the reference template stored in the memory to determine a difference, wherein the difference is computed as a waveform similarity coefficient, wherein the waveform similarity coefficient is determined by comparing the value of the test aspect of the inputted test portion with the value of the reference aspect of the reference template to compute a waveform similarity coefficient value between 0 and 1, wherein a value of zero for the waveform similarity coefficient indicates no similarity between the inputted test portion and the reference template, and a value of 1 for the waveform similarity coefficient indicates complete similarity between the inputted test portion and the reference template, and
cause the output device to output an alert responsive to the waveform similarity coefficient being below a similarity threshold, wherein the similarity threshold comprises a selected value between 0 and 1,
**characterized in that** the wearable medical system further comprises:
a biasing mechanism for biasing the ECG electrodes towards the patient's body responsive to a monitoring condition being met, the monitoring condition comprising the waveform similarity coefficient being below the similarity threshold, and for otherwise not biasing the ECG electrodes towards the patient's body.

2. The WM system of claim 1, in which the processor is further configured to
generate a composite amplitude waveform from early amplitude waveforms of respective ones of the early portions of the ECG signal, and
store the composite amplitude waveform in the memory as the reference template.

3. The WM system of claim 2, in which
the processor is further configured to identify similarity statistics of early amplitude waveforms of respective ones of the early portions of the ECG signal, and
in which the composite amplitude waveform is generated from those of the early amplitude waveforms whose similarity statistics exceed a threshold.

4. The WM system of claim 2 or 3, in which
the processor is further configured to align the early amplitude waveforms, before generating the composite amplitude waveform from them,
wherein the processor is further configured to filter the early amplitude waveforms prior to aligning them.

5. The WM system of any of the preceding claims, in which
a default template is stored in the memory before the reference template is made, and
the difference is determined by comparing the value of the test aspect of the inputted test portion with a value of a reference aspect of the default template instead of with the value of a reference aspect of the reference template,
wherein the default template was constructed from prior data of prior patients,
preferably prior patients who match the ambulatory patient in at least one demographic parameter of a set that includes gender, age, height and weight.

6. The WM system of any of the preceding claims, in which
the processor is further configured to determine whether a monitoring condition is met, the monitoring condition comprising the waveform similarity coefficient being below the similarity threshold, and
the test portion is input responsive to determining that the monitoring condition is met,
wherein the processor further includes a clock configured to keep a time, and the monitoring condition is met responsive to the time reaching a checking moment.

7. The WM system of claim 6, in which the monitoring condition includes that a heart rate of the patient is above a heart rate threshold.

8. The WM system of claim 6 or 7, further comprising:
a monitoring device configured to monitor at least one physiological parameter of the ambulatory patient that is not the ECG signal of the ambulatory patient; and
the monitoring condition is met responsive to a value of the monitored parameter reaching a monitoring threshold,
wherein the WM system further comprises a user interface (UI) having an input device that is configured to be actuated by the ambulatory patient, and in which the monitoring condition is met responsive to the ambulatory patient actuating the input device.

9. The WM system of claim 8, in which the physiological parameter is one chosen from the ambulatory patient's heart rate, blood perfusion, blood flow, blood pressure, blood oxygen level, pulsatile change in light transmission or reflection properties of perfused tissue, heart sounds, breathing sounds, pulse and motion.

10. The WM system of any of the preceding claims, in which the test aspect includes an aggregated waveform of the test portion of the ECG signal.

11. The WM system of any of the preceding claims, in which the WM system includes a user interface (UI), the output device belongs to the UI, and the alert is output by the output device emitting a human-perceptible indication.

12. The WM system of any of the preceding claims, in which the WM system includes a communication module that is the output device, and the alert is output by the communication module transmitting a notification to another device.

13. The WM system of any of the preceding claims, in which
the test aspect includes a QRS interval of a QRS complex of the inputted test portion of the ECG signal, and/or
the test aspect includes an ST segment after a QRS complex of the inputted test portion of the ECG signal, and/or
the test aspect includes an ST interval of a QRS complex of the inputted test portion of the ECG signal, and/or
the test aspect includes a T-wave after a QRS complex of the inputted test portion of the ECG signal.

14. A method for a wearable medical (WM) system, the WM system including electrocardiogram (ECG) electrodes, a support structure configured to be worn by an ambulatory patient so as to maintain the ECG electrodes on a body of the ambulatory patient, the ECG electrodes configured, when thus maintained, to sense an ECG signal of the ambulatory patient, an output device, and a memory, the method comprising:
storing a reference template in the memory, the reference template made from one or more early portions of the ECG signal of the ambulatory patient that are sensed during a first time duration;
inputting a test portion of the ECG signal of the ambulatory patient that is sensed during a second time duration;
comparing a value of a test aspect of the inputted test portion with a value of a reference aspect of the reference template stored in the memory to determine a difference **characterized in that** the difference is computed as a waveform similarity coefficient, wherein the waveform similarity coefficient is determined by comparing the value of the test aspect of the inputted test portion with the value of the reference aspect of the reference template to compute a waveform similarity coefficient value between 0 and 1, wherein a value of zero for the waveform similarity coefficient indicates no similarity between the inputted test portion and the reference template, and a value of 1 for the waveform similarity coefficient indicates complete similarity between the inputted test portion and the reference template;
outputting, by the output device, an alert responsive to the waveform similarity coefficient being below a similarity threshold, wherein the similarity threshold comprises a selected value between 0 and 1; and.
biasing the ECG electrodes towards the patient's body by means of a biasing mechanism responsive to a monitoring condition being met, the monitoring condition comprising the waveform similarity coefficient being below the similarity threshold, and for otherwise not biasing the ECG electrodes towards the patient's body.

## Patentansprüche

1. Tragbares medizinisches System (WM-System (wearable medical system)) für einen gehfähigen Patienten, mit:
Elektrokardiogramm-Elektroden (EKG-Elektroden),
einer Tragstruktur, die so ausgebildet ist, dass sie von dem gehfähigen Patienten so getragen wird, dass die EKG-Elektroden an einem Körper des gehfähigen Patienten gehalten werden, wobei die EKG-Elektroden so ausgebildet sind, dass sie dann, wenn sie so gehalten werden, ein EKG-Signal des gehfähigen Patienten erfassen,
einer Ausgabevorrichtung,
einem Speicher, der so ausgebildet ist, dass eine Referenzvorlage darin abgelegt ist, wobei die Referenzvorlage aus einem oder mehreren frühen Abschnitten des EKG-Signals des gehfähigen Patienten besteht, die während einer ersten Zeitdauer erfasst werden, und
einem Prozessor, der so ausgebildet ist, dass er:
einen Prüfabschnitt des EKG-Signals des gehfähigen Patienten einliest, der während einer zweiten Zeitdauer erfasst wird,
einen Wert eines Prüfaspekts des eingelesenen Prüfabschnitts mit einem Wert eines Referenzaspekts der im Speicher abgelegten Referenzvorlage vergleicht, um eine Differenz zu ermitteln, wobei die Differenz als Wellenform-Ähnlichkeitskoeffizient berechnet wird, wobei der Wellenform-Ähnlichkeitskoeffizient ermittelt wird, indem der Wert des Prüfaspekts des eingelesenen Prüfabschnitts mit dem Wert des Referenzaspekts der Referenzvorlage verglichen wird, um einen Wellenform-Ähnlichkeitskoeffizientenwert zwischen 0 und 1 zu berechnen, wobei ein Wert von Null für den Wellenform-Ähnlichkeitskoeffizienten angibt, dass keine Ähnlichkeit zwischen dem eingelesenen Prüfabschnitt und der Referenzvorlage vorliegt, und ein Wert von 1 für den Wellenform-Ähnlichkeitskoeffizienten eine vollständige Ähnlichkeit zwischen dem eingelesenen Prüfabschnitt und der Referenzvorlage angibt, und
bewirkt, dass die Ausgabevorrichtung als Reaktion darauf, dass der Wellenform-Ähnlichkeitskoeffizient unterhalb eines Ähnlichkeitsschwellenwertes liegt, ein Warnsignal ausgibt, wobei der Ähnlichkeitsschwellenwert einen ausgewählten Wert zwischen 0 und 1 umfasst,
**dadurch gekennzeichnet, dass** das tragbare medizinische System ferner Folgendes umfasst:
einen Vorspannmechanismus, der als Reaktion darauf, dass eine Überwachungsbedingung erfüllt ist, die EKG-Elektroden zum Körper des Patienten hin vorspannt, wobei die Überwachungsbedingung beinhaltet, dass der Wellenform-Ähnlichkeitskoeffizient unterhalb des Ähnlichkeitsschwellenwertes liegt, und der anderenfalls die EKG-Elektroden nicht zum Körper des Patienten hin vorspannt.

2. WM-System nach Anspruch 1, bei dem der Prozessor ferner so ausgebildet ist, dass er
aus frühen Amplitudenwellenformen jeweiliger der frühen Abschnitte des EKG-Signals eine zusammengesetzte Amplitudenwellenform erzeugt und
die zusammengesetzte Amplitudenwellenform als Referenzvorlage im Speicher ablegt.

3. WM-System nach Anspruch 2, bei dem
der Prozessor ferner so ausgebildet ist, dass er Ähnlichkeitsstatistiken früher Amplitudenwellenformen jeweiliger der frühen Abschnitte des EKG-Signals erkennt, und
bei dem die zusammengesetzte Amplitudenwellenform aus denjenigen der frühen Amplitudenwellenformen erzeugt wird, deren Ähnlichkeitsstatistiken einen Schwellenwert überschreiten.

4. WM-System nach Anspruch 2 oder 3, bei dem
der Prozessor ferner so ausgebildet ist, dass er die frühen Amplitudenwellenformen ausrichtet, bevor er aus ihnen die zusammengesetzte Amplitudenwellenform erzeugt,
wobei der Prozessor ferner so ausgebildet ist, dass er die frühen Amplitudenwellenformen filtert, bevor er sie ausrichtet.

5. WM-System nach einem der vorhergehenden Ansprüche, bei dem
eine Standardvorlage im Speicher abgelegt wird, bevor die Referenzvorlage erstellt wird, und
die Differenz ermittelt wird, indem der Wert des Prüfaspekts des eingelesenen Prüfabschnitts mit einem Wert eines Referenzaspekts der Standardvorlage anstatt mit dem Wert eines Referenzaspekts der Referenzvorlage verglichen wird,
wobei die Standardvorlage aus vorherigen Daten vorheriger Patienten aufgebaut wurde,
vorzugsweise vorheriger Patienten, die in wenigstens einem demographischen Parameter eines Satzes, zu dem Geschlecht, Alter, Größe und Gewicht gehören, mit dem gehfähigen Patienten übereinstimmen.

6. WM-System nach einem der vorhergehenden Ansprüche, bei dem
der Prozessor ferner so ausgebildet ist, dass er ermittelt, ob eine Überwachungsbedingung erfüllt ist, wobei die Überwachungsbedingung beinhaltet, dass der Wellenform-Ähnlichkeitskoeffizient unterhalb des Ähnlichkeitsschwellenwerts liegt, und
der Prüfabschnitt als Reaktion auf die Feststellung, dass die Überwachungsbedingung erfüllt ist, eingelesen wird,
wobei der Prozessor ferner einen Taktgeber aufweist, der dazu ausgebildet ist, eine Zeit zu messen, und die Überwachungsbedingung als Reaktion darauf erfüllt ist, dass die Zeit einen Überprüfungszeitpunkt erreicht.

7. WM-System nach Anspruch 6, bei dem die Überwachungsbedingung beinhaltet, dass eine Herzfrequenz des Patienten oberhalb eines Herzfrequenzschwellenwerts liegt.

8. WM-System nach Anspruch 6 oder 7, ferner mit:
einer Überwachungsvorrichtung, die so ausgebildet ist, dass sie wenigstens einen physiologischen Parameter des gehfähigen Patienten überwacht, bei dem es sich nicht um das EKG-Signal des gehfähigen Patienten handelt, und
die Überwachungsbedingung als Reaktion darauf erfüllt ist, dass ein Wert des überwachten Parameters einen Überwachungsschwellenwert erreicht,
wobei das WM-System ferner eine Benutzerschnittstelle (Ul) mit einer Eingabevorrichtung umfasst, die zur Betätigung durch den gehfähigen Patienten ausgebildet ist, und bei dem die Überwachungsbedingung als Reaktion darauf erfüllt ist, dass der gehfähige Patient die Eingabevorrichtung betätigt.

9. WM-System nach Anspruch 8, bei dem der physiologische Parameter einer ist, der aus der Herzfrequenz, der Blutperfusion, dem Blutfluss, dem Blutdruck, dem Blutsauerstoffgehalt, der pulsierenden Veränderung der Lichtdurchlässigkeits- oder -reflexionseigenschaften von durchblutetem Gewebe, den Herztönen, den Atemgeräuschen, dem Puls und der Bewegung des gehfähigen Patienten ausgewählt ist.

10. WM-System nach einem der vorhergehenden Ansprüche, bei dem der Prüfaspekt eine aggregierte Wellenform des Prüfabschnitts des EKG-Signals aufweist.

11. WM-System nach einem der vorhergehenden Ansprüche, wobei das WM-System eine Benutzerschnittstelle (UI) aufweist, die Ausgabevorrichtung zu der UI gehört und das Warnsignal ausgegeben wird, indem die Ausgabevorrichtung einen vom Menschen wahrnehmbaren Hinweis aussendet.

12. WM-System nach einem der vorhergehenden Ansprüche, wobei das WM-System ein Kommunikationsmodul aufweist, bei dem es sich um die Ausgabevorrichtung handelt, und das Warnsignal ausgegeben wird, indem das Kommunikationsmodul eine Benachrichtigung an eine andere Vorrichtung sendet.

13. WM-System nach einem der vorhergehenden Ansprüche, bei dem
der Prüfaspekt ein QRS-Intervall eines QRS-Komplexes des eingelesenen Prüfabschnitts des EKG-Signals enthält und/oder
der Prüfaspekt eine ST-Strecke nach einem QRS-Komplex des eingelesenen Prüfabschnitts des EKG-Signals enthält und/oder
der Prüfaspekt ein ST-Intervall eines QRS-Komplexes des eingelesenen Prüfabschnitts des EKG-Signals enthält und/oder
der Prüfaspekt eine T-Welle nach einem QRS-Komplex des eingelesenen Prüfabschnitts des EKG-Signals enthält.

14. Verfahren für ein tragbares medizinisches System (WM-System (wearable medical system)), wobei das WM-System Elektrokardiogramm-Elektroden (EKG-Elektroden), eine Tragstruktur, die so ausgebildet ist, dass sie von einem gehfähigen Patienten so getragen wird, dass die EKG-Elektroden an einem Körper des gehfähigen Patienten gehalten werden, wobei die EKG-Elektroden so ausgebildet sind, dass sie dann, wenn sie so gehalten werden, ein EKG-Signal des gehfähigen Patienten erfassen, eine Ausgabevorrichtung und einen Speicher aufweist, wobei das Verfahren Folgendes umfasst:
eine Referenzvorlage wird im Speicher abgelegt, wobei die Referenzvorlage aus einem oder mehreren frühen Abschnitten des EKG-Signals des gehfähigen Patienten besteht, die während einer ersten Zeitdauer erfasst werden,
ein Prüfabschnitts des EKG-Signals des gehfähigen Patienten, der während einer zweiten Zeitdauer erfasst wird, wird eingelesen,
ein Wert eines Prüfaspekts des eingelesenen Prüfabschnitts wird mit einem Wert eines Referenzaspekts der im Speicher abgelegten Referenzvorlage verglichen, um eine Differenz zu ermitteln, **dadurch gekennzeichnet, dass** die Differenz als Wellenform-Ähnlichkeitskoeffizient berechnet wird, wobei der Wellenform-Ähnlichkeitskoeffizient ermittelt wird, indem der Wert des Prüfaspekts des eingelesenen Prüfabschnitts mit dem Wert des Referenzaspekts der Referenzvorlage verglichen wird, um einen Wellenform-Ähnlichkeitskoeffizientenwert zwischen 0 und 1 zu berechnen, wobei ein Wert von Null für den Wellenform-Ähnlichkeitskoeffizienten angibt, dass keine Ähnlichkeit zwischen dem eingelesenen Prüfabschnitt und der Referenzvorlage vorliegt, und ein Wert von 1 für den Wellenform-Ähnlichkeitskoeffizienten eine vollständige Ähnlichkeit zwischen dem eingelesenen Prüfabschnitt und der Referenzvorlage angibt,
von der Ausgabevorrichtung wird als Reaktion darauf, dass der Wellenform-Ähnlichkeitskoeffizient unterhalb eines Ähnlichkeitsschwellenwertes liegt, ein Warnsignal ausgegeben, wobei der Ähnlichkeitsschwellenwert einen ausgewählten Wert zwischen 0 und 1 umfasst, und
die EKG-Elektroden werden als Reaktion darauf, dass eine Überwachungsbedingung erfüllt ist, mittels eines Vorspannmechanismus zum Körper des Patienten hin vorgespannt, wobei die Überwachungsbedingung beinhaltet, dass der Wellenform-Ähnlichkeitskoeffizient unterhalb des Ähnlichkeitsschwellenwertes liegt, und anderenfalls werden die EKG-Elektroden nicht zum Körper des Patienten hin vorgespannt.

## Revendications

1. Système médical portatif (système WM (wearable medical system)) pour un patient ambulatoire, comprenant :
des électrodes d'électrocardiogramme (ECG) ;
une structure de support réalisée de manière à être portée par le patient ambulatoire afin de maintenir les électrodes ECG sur un corps du patient ambulatoire, les électrodes ECG étant réalisées, lorsqu'elles sont ainsi maintenues, de manière à détecter un signal ECG du patient ambulatoire ;
un dispositif de sortie ;
une mémoire réalisée de manière à stocker un modèle de référence, le modèle de référence étant établi à partir d'une ou de plusieurs parties initiales du signal ECG du patient ambulatoire qui sont détectées pendant une première période de temps ; et
un processeur réalisé de manière :
à saisir une partie de test du signal ECG du patient ambulatoire détectée pendant une deuxième période de temps,
à comparer une valeur d'un aspect de test de la partie de test saisie à une valeur d'un aspect de référence du modèle de référence stocké dans la mémoire afin de déterminer une différence, la différence étant calculée sous forme de coefficient de similarité de formes d'onde, le coefficient de similarité de formes d'onde étant déterminé en comparant la valeur de l'aspect de test de la partie de test saisie à la valeur de l'aspect de référence du modèle de référence afin de calculer une valeur de coefficient de similarité de formes d'onde comprise entre 0 et 1, une valeur égale à zéro pour le coefficient de similarité de formes d'onde indiquant l'absence de similarité entre la partie de test saisie et le modèle de référence, et une valeur de 1 pour le coefficient de similarité de formes d'onde indiquant une similarité totale entre la partie de test saisie et le modèle de référence, et
à amener le dispositif de sortie à émettre une alerte en réponse au fait que le coefficient de similarité de formes d'onde est inférieur à un seuil de similarité, le seuil de similarité comprenant une valeur sélectionnée comprise entre 0 et 1,
**caractérisé en ce que** le système médical portatif comprend en outre :
un mécanisme de précontrainte pour précontraindre les électrodes ECG vers le corps du patient en réponse au fait qu'une condition de surveillance est remplie, la condition de surveillance comprenant le fait que le coefficient de similarité de formes d'onde est inférieur au seuil de similarité, et ne pas précontraindre les électrodes ECG vers le corps du patient dans le cas contraire.

2. Système WM selon la revendication 1, le processeur étant en outre réalisé de manière
à générer une forme d'onde d'amplitude composée à partir de formes d'onde d'amplitude initiales de parties respectives parmi les parties initiales du signal ECG, et
à stocker la forme d'onde d'amplitude composée dans la mémoire en tant que modèle de référence.

3. Système WM selon la revendication 2,
le processeur étant en outre réalisé de manière à identifier des statistiques de similarité de formes d'onde d'amplitude initiales de parties respectives parmi les parties initiales du signal ECG, et
la forme d'onde d'amplitude composée étant générée à partir de celles des formes d'onde d'amplitude initiales dont les statistiques de similarité dépassent un seuil.

4. Système WM selon la revendication 2 ou 3,
le processeur étant en outre réalisé de manière à aligner les formes d'onde d'amplitude initiales avant de générer la forme d'onde d'amplitude composée à partir de celles-ci,
le processeur étant en outre réalisé de manière à filtrer les formes d'onde d'amplitude initiales avant de les aligner.

5. Système WM selon l'une des revendications précédentes,
un modèle par défaut étant stocké dans la mémoire avant la création du modèle de référence, et
la différence étant déterminée en comparant la valeur de l'aspect de test de la partie de test saisie à une valeur d'un aspect de référence du modèle par défaut plutôt qu'à la valeur d'un aspect de référence du modèle de référence,
le modèle par défaut ayant été construit à partir de données antérieures de patients antérieurs,
de préférence de patients antérieurs correspondant au patient ambulatoire pour au moins un paramètre démographique d'un ensemble comprenant le sexe, l'âge, la taille et le poids.

6. Système WM selon l'une des revendications précédentes,
le processeur étant en outre réalisé de manière à déterminer si une condition de surveillance est remplie, la condition de surveillance comprenant le fait que le coefficient de similarité de formes d'onde est inférieur au seuil de similarité, et
la partie de test étant saisie en réponse à la détermination que la condition de surveillance est remplie,
le processeur comprenant en outre une horloge réalisée de manière à maintenir un cycle, et la condition de surveillance étant remplie en réponse au fait que le cycle atteint un moment de vérification.

7. Système WM selon la revendication 6, la condition de surveillance comprenant le fait qu'une fréquence cardiaque du patient est supérieure à un seuil de fréquence cardiaque.

8. Système WM selon la revendication 6 ou 7, comprenant en outre :
un dispositif de surveillance réalisé de manière à surveiller au moins un paramètre physiologique du patient ambulatoire autre que le signal ECG du patient ambulatoire ; et
la condition de surveillance étant remplie lorsqu'une valeur du paramètre surveillé atteint un seuil de surveillance,
le système WM comprenant en outre une interface utilisateur (Ul) présentant un dispositif d'entrée réalisé de manière à être actionné par le patient ambulatoire, et la condition de surveillance étant remplie lorsque le patient ambulatoire actionne le dispositif d'entrée.

9. Système WM selon la revendication 8, le paramètre physiologique étant l'un choisi parmi la fréquence cardiaque du patient ambulatoire, la perfusion sanguine, le débit sanguin, la pression artérielle, le taux d'oxygène dans le sang, la variation pulsatile des propriétés de transmission ou de réflexion de la lumière d'un tissu perfusé, les bruits cardiaques, les bruits respiratoires, le pouls et le mouvement.

10. Système WM selon l'une des revendications précédentes, l'aspect de test comprenant une forme d'onde agrégée de la partie de test du signal ECG.

11. Système WM selon l'une des revendications précédentes, le système WM présentant une interface utilisateur (UI), le dispositif de sortie faisant partie de l'UI, et l'alerte étant émise en ce que le dispositif de sortie émet une indication perceptible par l'humain.

12. Système WM selon l'une des revendications précédentes, le système WM comprenant un module de communication qui est le dispositif de sortie, et l'alerte étant émise en ce que le module de communication transmet une notification à un autre dispositif.

13. Système WM selon l'une des revendications précédentes,
l'aspect de test comprenant un intervalle QRS d'un complexe QRS de la partie de test saisie du signal ECG, et/ou
l'aspect de test comprenant un segment ST après un complexe QRS de la partie de test saisie du signal ECG, et/ou
l'aspect de test comprenant un intervalle ST d'un complexe QRS de la partie de test saisie du signal ECG, et/ou
l'aspect de test comprenant une onde T après un complexe QRS de la partie de test saisie du signal ECG.

14. Procédé pour un système médical portatif (système WM (wearable medical system)), le système WM comprenant des électrodes d'électrocardiogramme (ECG), une structure de support réalisée de manière à être portée par un patient ambulatoire de manière à maintenir les électrodes ECG sur un corps du patient ambulatoire, les électrodes ECG étant réalisées, lorsqu'elles sont ainsi maintenues, de manière à détecter un signal ECG du patient ambulatoire, un dispositif de sortie et une mémoire, le procédé comprenant :
le stockage d'un modèle de référence dans la mémoire, le modèle de référence étant établi à partir d'une ou de plusieurs parties initiales du signal ECG du patient ambulatoire qui sont détectées pendant une première période de temps ;
l'entrée d'une partie de test du signal ECG du patient ambulatoire qui est détectée pendant une deuxième période de temps ;
la comparaison d'une valeur d'un aspect de test de la partie de test saisie à une valeur d'un aspect de référence du modèle de référence stocké dans la mémoire afin de déterminer une différence, **caractérisé en ce que** la différence est calculée sous forme de coefficient de similarité de formes d'onde, le coefficient de similarité de formes d'onde étant déterminé en comparant la valeur de l'aspect de test de la partie de test saisie à la valeur de l'aspect de référence du modèle de référence afin de calculer une valeur de coefficient de similarité de formes d'onde comprise entre 0 et 1, une valeur égale à zéro pour le coefficient de similarité de formes d'onde indiquant l'absence de similarité entre la partie de test saisie et le modèle de référence, et une valeur de 1 pour le coefficient de similarité de formes d'onde indiquant une similarité totale entre la partie de test saisie et le modèle de référence ;
l'émission, par le dispositif de sortie, d'une alerte en réponse au fait que le coefficient de similarité de formes d'onde est inférieur à un seuil de similarité, le seuil de similarité comprenant une valeur sélectionnée comprise entre 0 et 1 ; et
la précontrainte, au moyen d'un mécanisme de précontrainte, des électrodes ECG vers le corps du patient en réponse au fait qu'une condition de surveillance est remplie, la condition de surveillance comprenant le fait que le coefficient de similarité de formes d'onde est inférieur au seuil de similarité, et, dans le cas contraire, pas de précontrainte des électrodes ECG vers le corps du patient.
